# EUROPEAN PATENT SPECIFICATION

(11) **EP 3 368 527 B1**
(45) Date of publication and mention of the grant of the patent: **28.04.2021**
(21) Application number: 16859141.0
(22) Date of filing: 26.10.2016
(51) Int. Cl.: C07D 471/04, A61K 31/519, A61P 35/00

(54) **PALBOCICLIB TOSYLATE**
PALBOCICLIB-TOSYLAT
TOSYLATE DE PALBOCICLIB

(30) Priority: 28.10.2015 HU 1500506
(43) Date of publication of application: 05.09.2018
(73) Proprietor: Egis Gyógyszergyár Zrt., 1106 Budapest (HU)
(72) Inventor: LUKÁCS, Gyula, 1163 Budapest (HU); MÁRVÁNYOS, Ede László, 1114 Budapest (HU); BERECZ, Gábor, 1149 Budapest (HU); HÉDER, János Levente, 2527 Máriahalom (HU); MILEN, Mátyás, 1144 Budapest (HU); PEREGI, Balázs, 2013 Pomáz (HU); GUDOR, Róbert, 8700 Marcali (HU); VOLK, Balázs, 1106 Budapest (HU); TÓTHNÉ LAURITZ, Mária, 1042 Budapest (HU)
(74) Representative: Stolmár & Partner Patentanwälte PartG mbB
(86) International application number: PCT/HU2016/050050
(87) International publication number: WO 2017/072543

(56) References cited:
- EP-A1- 3 231 805
- WO-A1-2005/005426
- WO-A1-2016/024249
- WO-A1-2016/066420
- WO-A1-2016/127963
- CN-A- 104 327 078

## Description

### The subject of the invention

Our invention relates to various new salts of palbociclib, to their anhydrous forms, their hydrates and solvates, to their production, and to medical preparations containing these, as well as to the medical use of the above forms.

The structural formula of 6-acetyl-8-cyclopentyl-5-methyl-2-{[5-(1-piperazinyl)-2-piridinyl]amino}pyrido[2,3-*d*]pyrimidin-7(8*H*)-one, international non-proprietary name: palbociclib (1) [CAS: 571190-30-2] is the following:

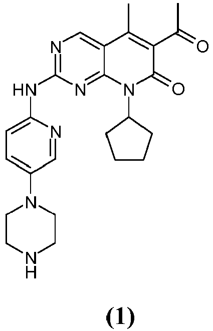

### The state of the art

It is known that palbociclib selectively blocks two important cellular enzymes of cell cycle regulation responsible for the growth and proliferation of tumour cells, types 4 and 6 cyclin-dependent kinases (CDK4 and CDK6).

Palbociclib offers a progressive therapy opportunity for breast cancer patients in an advanced stage. Palbociclib therapy supplemented with letrozole significantly improves the progression free survival of ER⁺/HER⁻ breast cancer patients judged to be suitable for the treatment as compared with letrozole monotherapy.

International patent application number WO 2003/062236 describes formula (1) palbociclib and its hydrochloric acid salt. The stoichiometry of the hydrochloric acid salt (example 36) described in the application: palbociclib· 2.4 H₂O · 1.85 HCl.

It is stated in international patent application number WO 2005/005426 that it is necessary to produce salts due to the lower solubility of the base (9 µg/ml). The application presents palbociclib isethionate Form A, isethionate Form B and isethionate Form D. The application also describes palbociclib mono-mesylate Form A, Form B, Form C and Form D, a monohydrochloride, a di-hydrochloride and a tosylate salt form. On the basis of that stated in the application the palbociclib di-HCI salt shows suitable water solubility, but it is also able to absorb a large amount of water (>2 m/m %) even at low relative humidity (10% RH), therefore it is unsuitable for the purpose of developing pharmaceutical preparations. Furthermore, on the basis of that stated, the presented palbociclib mono-HCl salt form is also unsuitable for medicine development purposes, as at relative humidity above 80% it is able to absorb more than 2% of water, in addition problems occurred with its production during the scale-up process.

International patent application publication number WO 2014/128588 describes the Form A and Form B crystalline palbociclib bases. It describes the Form A produced as having better physical-chemical properties, its grain size is characterised with the parameters D₁₀: 5-10 µm, D₅₀: 10-45 µm, D₉₀: 30-125 µm as compared to the base produced in the basic patent.

WO 2016/066420 A1 discloses crystalline forms of mono-HCl salt of palbociclib as well as methods for the preparation thereof.

EP 3 231 805 A1 discloses a crystalline form of the maleate salt of palbociclib.

### The objective of the invention

There is a constant demand in the pharmaceuticals industry to be able to reproducibly manufacture pure and morphologically homogenous active substances. This is the basic condition of being jointly able to satisfy the pharmaceutical formulation, quality assurance and registration authority requirements. It is well known that in the case of various salts and polymorphs differences occur in important attributes such as solubility, chemical stability, polymorphic stability, dissolution rate, bioavailability, filterability, ability to dry, and form tablets. Furthermore from the point of view of the cost-effectiveness of production it is extremely important to produce the product with a method that may also be realised on the industrial scale, that is easy to reproduce and results in a morphologically homogenous, contaminant-frcc salt.

In aqueous medium palbociclib base has low solubility, which limits its bioavailability. The low solubility also limits the method of administration of the compound and its formulation as a solid medicine.

Therefore, there is a demand for the production of new salts of formula (1) palbociclib base that have preferable properties as compared to the base and the salts produced to date. Therefore, the objective of the elaboration of our invention was the production of high-purity, homogenous morphology, new palbociclib salts the stability and physical-chemical properties of which are more preferable than those of the base and the known salts, which have chemical stability that is at least as good as that of the palbociclib base or the known salts, and furthermore, which may be reproducibly produced and produced on industrial scales.

The above objective was realised with the production of the new salts and new polymorphs of palbociclib according to our invention, more specifically with the production of salts of palbociclib formed with 4-toluenesulfonic acid.

The idea forming the basis of the invention is the production of new palbociclib salts that have better solubility in an aqueous medium than palbociclib base, and that have preferable stability and physical-chemical properties than those of the palbociclib salts characterised in earlier patent applications and of the palbociclib base.

It was surprising to experience that the new palbociclib salts forming the subject of the invention have better solubility under certain conditions as compared to the solubility data of the base described in the literature. A description of the solubility test is presented in our invention after the presentation of the morphological data.

Among the palbociclib salts according to the invention, the palbociclib tosylate salt form proved to be especially preferable, as this is, on the one part, more stable than the reference palbociclib forms, i.e. fewer contaminants are formed in them during storage, for example, and, on the other part, it has significantly better solubility in a slightly acidic (pH=4.5) environment. Additionally, this salt forms has surprisingly low hygroscopicity.

### Figures

Figure 1: the palbociclib hydrogen bromide (1:1) salt (not covered by the invention) x-ray powder diffractogram
Figure 2: the palbociclib hydrogen bromide (1:1) salt (not covered by the invention) water sorption isotherms at 25 °C
Figure 3: the palbociclib hydrogen bromide (1:2) dihydrate salt (not covered by the invention) x-ray powder diffractogram
Figure 4: the palbociclib hydrogen bromide (1:2) dihydrate salt (not covered by the invention) sorption isotherms at 25 °C
Figure 5: the palbociclib hydrogen chloride (1:1) salt Form E (not covered by the invention) x-ray powder diffractogram
Figure 6: the palbociclib hydrogen chloride (1:1) salt Form E (not covered by the invention) water sorption isotherms at 25 °C
Figure 7: the palbociclib sulphate (2:1) salt dihydrate (not covered by the invention) x-ray powder diffractogram
Figure 8: the palbociclib sulphate (2:1) salt dihydrate (not covered by the invention) water sorption isotherms at 25 °C
Figure 9: the palbociclib camsylate (1:1) salt (not covered by the invention) x-ray powder diffractogram
Figure 10: the palbociclib camsylate (1:1) salt (not covered by the invention) water sorption isotherms at 25 °C
Figure 11: the palbociclib napsylate (1:1) salt (not covered by the invention) x-ray powder diffractogram
Figure 12: the palbociclib napsylate (1:1) salt (not covered by the invention) water sorption isotherms at 25 °C
Figure 13: the palbociclib napsylate (1:2) dihydrate salt (not covered by the invention) x-ray powder diffractogram
Figure 14: the palbociclib napsylate (1:2) dihydrate salt (not covered by the invention) water sorption isotherms at 25 °C
Figure 15: the palbociclib tosylate (1:1) salt x-ray powder diffractogram
Figure 16: the palbociclib tosylate (1:1) salt water sorption isotherms at 25 °C
Figure 17: the palbociclib citrate (1:1) monohydrate salt (not covered by the invention) x-ray powder diffractogram
Figure 18: the palbociclib citrate (1:1) monohydrate salt (not covered by the invention) water sorption isotherms at 25 °C
Figure 19: the palbociclib maleate (1:1) salt Form I (not covered by the invention) x-ray powder diffractogram
Figure 20: the palbociclib maleate (1:1) salt Form I (not covered by the invention) water sorption isotherms at 25 °C
Figure 21: the palbociclib maleate (1:1) salt Form II (not covered by the invention) x-ray powder diffractogram
Figure 22: the palbociclib maleate (1:1) salt Form II (not covered by the invention) water sorption isotherms at 25 °C
Figure 23: the palbociclib oxalate (1:1) salt (not covered by the invention) x-ray powder diffractogram
Figure 24: the palbociclib oxalate (1:1) salt (not covered by the invention) water sorption isotherms at 25 °C

### A detailed description of the invention

The disclosure relates to the normal and acid salts of palbociclib, as well as to the hydrate and solvate forms of these. The subject of the invention more specifically is:
- palbociclib tosylate (1:1) salt,

### NMR measurement conditions

The NMR spectra of the new salts according to our invention were recorded using the following devices:
- VARIAN INOVA 500 (500 MHz)
- BRUKER AVANCE III 400 (400 MHz)

The NMR measurements of the new salts were performed in all cases in the solution phase, deuterated dimethyl sulfoxide (DMSO-*d*₆) was used as the solvent.

**Dynamic vapour sorption analysis (DVS)**

| | | | |
|---|---|---|---|
| Device: | SMS DVS Advantage DVSA1-STD dynamic vapour sorption analyser | | |
| Atmosphere: | nitrogen | | |
| Total gas flow: | 200 ml/min | | |
| Solvent: | water | | |
| Temperature: | 25 °C | | |
| Regulation: | Open Loop | | |
| Sections: | DMDT | Step size: | 5% RH |
| | | Stability criterion: | 0.002 %/min |
| | | Window: | 5 min |
| | | Minimum stability time: | 30 min |
| | | Maximum section time: | 360 min |
| | | Data saving frequency: | 1 min |
| Measurement ranges: | Range 1: | actual % RH → 95% RH | |
| | Range 2: | 95% RH → 0% RH | |
| | Range 3: | 0% RH → actual % RH | |

### X-ray powder diffraction measurement conditions:

In the case of all substances described here the x-ray diffraction data recorded in the powder form of the new salts according to the invention were obtained under the following measurement conditions:

| | |
|---|---|
| Device: | PANalytical Empyrean X-ray powder diffractometer |
| Measuring operation mode: | Transmission |

**X-ray tube**

| | |
|---|---|
| Type: | Empyrean Long Fine Focus High Resolution tube |
| Anode: | Cu |
| Wavelength: | Kα (1.541874 Å) |
| Focussing: | line focus |

**Radiation source side optics**

| | |
|---|---|
| Divergence slit: | Fixed slit 1/2° |
| Mirror: | Elliptic focussing mirror |
| Soller slit: | 0.04 rad |
| Dispersion reducing slit: | Fixed slit 1/2° |

**Detector side optics**

| | |
|---|---|
| Dispersion inhibitor slit: | Programmable slit, in fixed mode: 1/2 ° |
| Soller slit: | 0.04 rad |

**Sample stage**

| | |
|---|---|
| Type: | Reflection-transmission spinner stage |
| Sample rotation speed: | 1 rps |
| Sample knife: | Using transmission radiation blocking |

**Detector**

| | |
|---|---|
| Type: | PIXcel 3D 1 × 1 area detector |
| Detection mode: | Scanning line detector (1D) mode |
| Active length: | 3.3473° |
| Sample preparation: | The un-pulverised samples were placed between Mylar sheets. |

**Measurement settings**

| | |
|---|---|
| Temperature: | room temperature |
| Accelerating voltage: | 45 kV |
| Anode heating current: | 40 mA |
| Scanning: | continuous gonio (θ/θ) scan |
| Measurement range: | 2.0000 - 34.9964 °2θ |
| Step gap: | 0.0131° 2θ |
| Step duration: | 109.650 s |
| No of measurement cycles: | 1 |
| Measurement time: | ∼20 min |

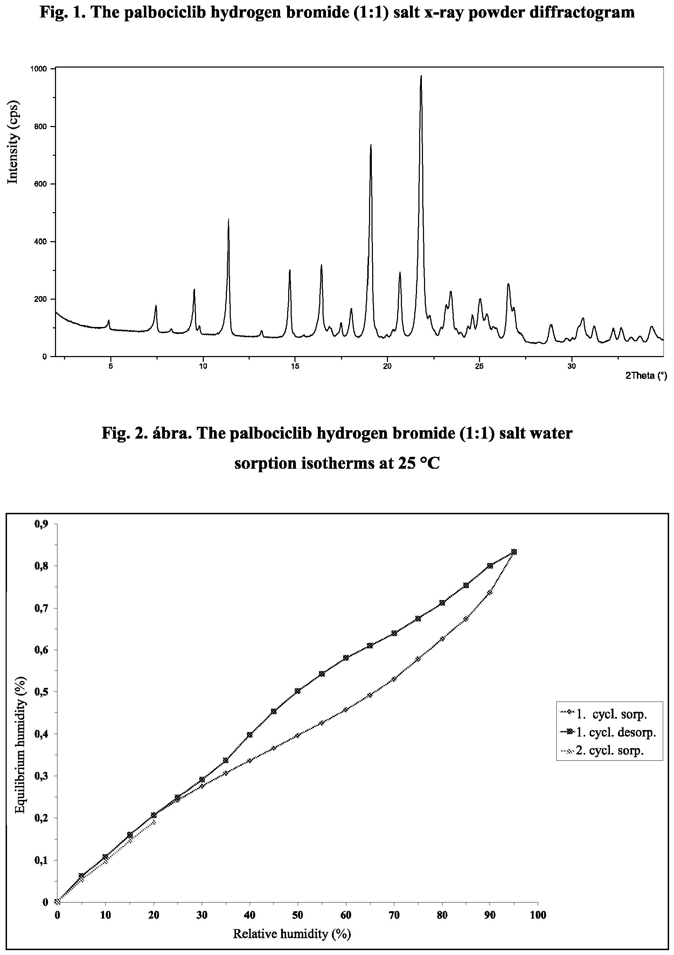

Disclosed herein is the crystalline palbociclib hydrogen bromide (1:1) salt form, the characteristic x-ray powder diffraction peaks of which are the following: 2θ (±0.2° 2θ): 7.46; 9.52; 11.39; 19.09; 21.82. More specifically it may be characterised with the following x-ray powder diffraction peaks: 2θ (±0.2° 2θ): 7.46; 9.52; 11.39; 14.71; 16.41; 19.09; 20.67; 21.82; 23.44; 25.02. Even more specifically it may be characterised with the following x-ray powder diffraction peaks: 2θ (±0.2° 2θ): 4.89; 7.46; 8.28; 9.52; 9.79; 11.39; 13.20; 14.71; 16.41; 16.85; 17.49; 18.04; 19.09; 20.29; 20.67; 21.82; 22.26; 22.86; 23.15; 23.44; 23.73; 24.36; 24.62; 25.02; 25.39; 25.73; 26.55; 26.86; 28.83; 29.69; 30.31; 30.58; 31.22; 32.25; 32.65; 33.19; 33.68; 34.34. Its characteristic x-ray powder diffractogram is shown in figure 1, and the 1% or higher intensity signals are summarised in the following table 1.

**Table 1**

| The x-ray powder diffraction data of the palbociclib hydrogen bromide (1:1) salt form (relative intensities ≥ 1%) | | | |
|---|---|---|---|
| Peak | 2θ (°) | d (Å) | Relative intensity (%) |
| 1 | 4.89 | 18.04 | 1 |
| 2 | 7.46 | 11.85 | 8 |
| 3 | 8.28 | 10.68 | 2 |
| 4 | 9.52 | 9.28 | 12 |
| 5 | 9.79 | 9.03 | 2 |
| 6 | 11.39 | 7.76 | 30 |
| 7 | 13.20 | 6.70 | 3 |
| 8 | 14.71 | 6.02 | 16 |
| 9 | 16.41 | 5.40 | 25 |
| 10 | 16.85 | 5.26 | 5 |
| 11 | 17.49 | 5.07 | 4 |
| 12 | 18.04 | 4.91 | 10 |
| 13 | 19.09 | 4.65 | 58 |
| 14 | 20.29 | 4.37 | 3 |
| 15 | 20.67 | 4.29 | 19 |
| 16 | 21.82 | 4.07 | 100 |
| 17 | 22.26 | 3.99 | 9 |
| 18 | 22.86 | 3.89 | 2 |
| 19 | 23.15 | 3.84 | 10 |
| 20 | 23.44 | 3.79 | 18 |
| 21 | 23.73 | 3.75 | 1 |
| 22 | 24.36 | 3.65 | 4 |
| 23 | 24.62 | 3.61 | 7 |
| 24 | 25.02 | 3.56 | 19 |
| 25 | 25.39 | 3.50 | 9 |
| 26 | 25.73 | 3.46 | 4 |
| 27 | 26.55 | 3.36 | 20 |
| 28 | 26.86 | 3.32 | 9 |
| 29 | 28.83 | 3.09 | 6 |
| 30 | 29.69 | 3.01 | 1 |
| 31 | 30.31 | 2.95 | 5 |
| 32 | 30.58 | 2.92 | 12 |
| 33 | 31.22 | 2.86 | 5 |
| 34 | 32.25 | 2.77 | 5 |
| 35 | 32.65 | 2.74 | 6 |
| 36 | 33.19 | 2.70 | 2 |
| 37 | 33.68 | 2.66 | 3 |
| 38 | 34.34 | 2.61 | 6 |

The water sorption isotherms of the palbociclib hydrogen bromide (1:1) salt disclosed herein recorded at 25 °C are presented in figure 2. It is clearly visible that it advantageously does not exhibit significant hygroscopicity.

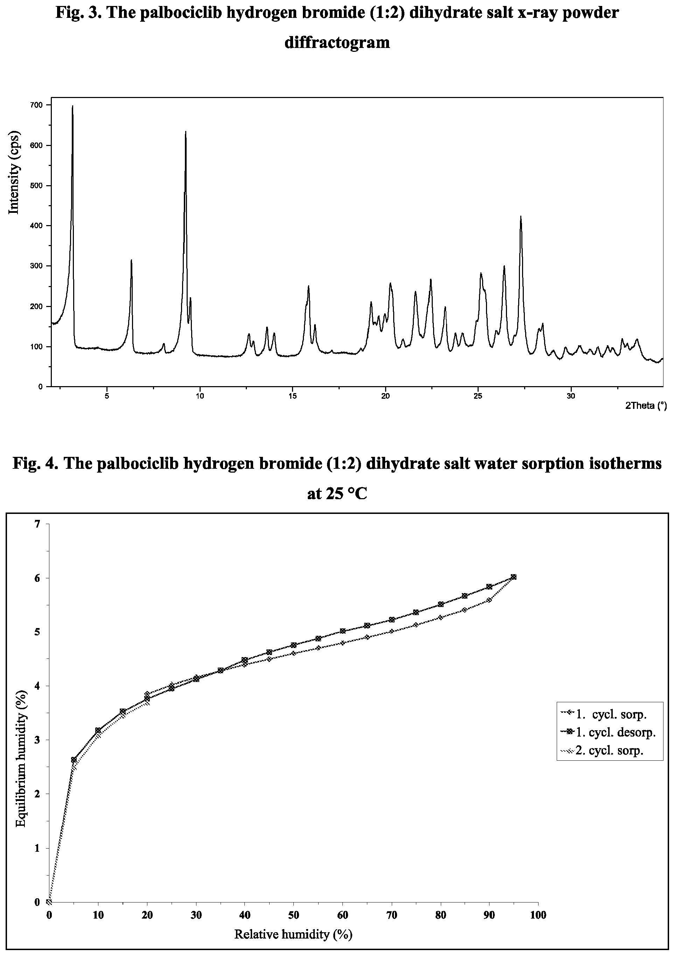

Disclosed herein is also the crystalline palbociclib hydrogen bromide (1:2) dihydrate salt form, the characteristic x-ray powder diffraction peaks of which are the following: 2θ (±0.2° 2θ): 3.16; 6.31; 9.22; 9.48; 27.30. More specifically it may be characterised with the following x-ray powder diffraction peaks: 2θ (±0.2° 2θ): 3.16; 6.31; 9.22; 9.48; 15.85; 19.18; 21.61; 22.44; 26.39; 27.30. Even more specifically it may be characterised with the following x-ray powder diffraction peaks: 2θ (±0,2° 2θ): 3.16; 6.31; 8.08; 9.22; 9.48; 12.63; 12.88; 13.62; 14.00; 15.85; 16.17; 19.18; 19.64; 19.96; 20.24; 20.93; 21.61; 22.44; 23.22; 23.76; 24.12; 24.90; 25.13; 25.39; 25.93; 26.39; 26.93; 27.30; 28.23; 28.47; 29.66; 30.46; 31.44; 31.98; 32.21; 32.74; 33.56. Its characteristic x-ray powder diffractogram is shown in figure 3, and the intensity signals greater than 4% are summarised in the following table 2:

**Table 2**

| The x-ray powder diffraction data of the palbociclib hydrogen bromide (1:2) dihydrate salt form (relative intensities ≥ 4%) | | | |
|---|---|---|---|
| peak | 2θ (°) | d (Å) | Relative intensity (%) |
| 1 | 3.16 | 27.95 | 100 |
| 2 | 6.31 | 13.99 | 35 |
| 3 | 8.08 | 10.93 | 5 |
| 4 | 9.22 | 9.58 | 78 |
| 5 | 9.48 | 9.32 | 20 |
| 6 | 12.63 | 7.00 | 9 |
| 7 | 12.88 | 6.87 | 6 |
| 8 | 13.62 | 6.50 | 11 |
| 9 | 14.00 | 6.32 | 11 |
| 10 | 15.85 | 5.59 | 22 |
| 11 | 16.17 | 5.48 | 13 |
| 12 | 19.18 | 4.62 | 29 |
| 13 | 19.64 | 4.52 | 18 |
| 14 | 19.96 | 4.44 | 23 |
| 15 | 20.24 | 4.38 | 28 |
| 16 | 20.93 | 4.24 | 10 |
| 17 | 21.61 | 4.11 | 38 |
| 18 | 22.44 | 3.96 | 61 |
| 19 | 23.22 | 3.83 | 30 |
| 20 | 23.76 | 3.74 | 12 |
| 21 | 24.12 | 3.69 | 19 |
| 22 | 24.90 | 3.57 | 13 |
| 23 | 25.13 | 3.54 | 33 |
| 24 | 25.39 | 3.50 | 39 |
| 25 | 25.93 | 3.43 | 11 |
| 26 | 26.39 | 3.37 | 54 |
| 27 | 26.93 | 3.31 | 7 |
| 28 | 27.30 | 3.26 | 72 |
| 29 | 28.23 | 3.16 | 16 |
| 30 | 28.47 | 3.13 | 13 |
| 31 | 29.66 | 3.01 | 7 |
| 32 | 30.46 | 2.93 | 10 |
| 33 | 31.44 | 2.84 | 5 |
| 34 | 31.98 | 2.80 | 8 |
| 35 | 32.21 | 2.78 | 6 |
| 36 | 32.74 | 2.73 | 10 |
| 37 | 33.56 | 2.67 | 18 |

The water sorption isotherms of the palbociclib hydrogen bromide (1:2) dihydrate salt disclosed herein recorded at 25 °C are presented in figure 4. It is clearly visible that the substance exhibits significant hygroscopicity.

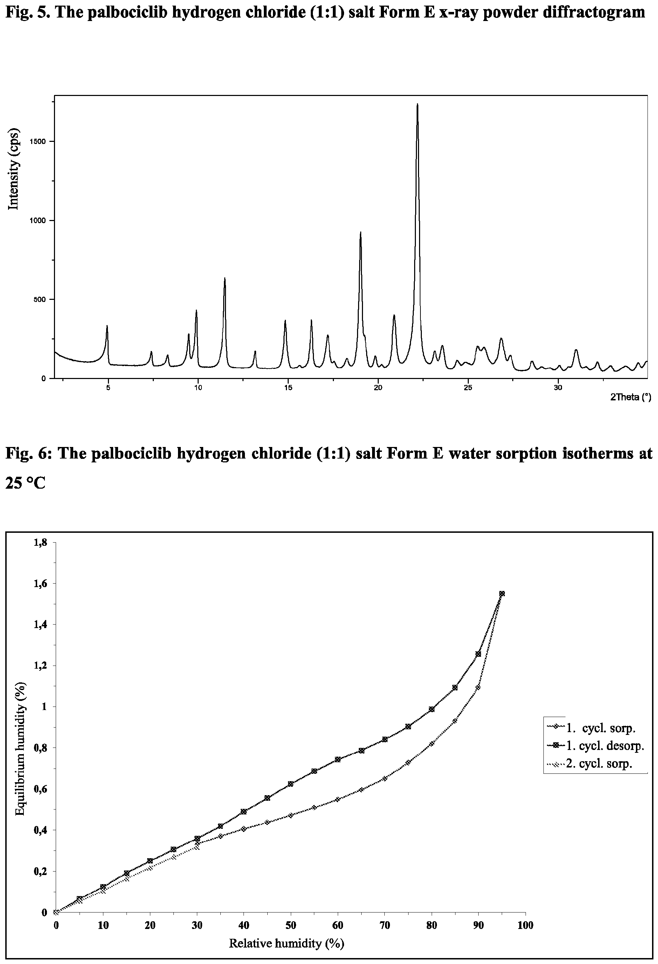

Disclosed herein is also the crystalline palbociclib hydrogen chloride (1:1) salt Form E, the characteristic x-ray powder diffraction peaks of which are the following: 2θ (±0,2° 2θ): 4.94; 9.88; 11.47; 19.01; 22.16. More specifically it may be characterised with the following x-ray powder diffraction peaks: 2θ (±0.2° 2θ): 4.94; 9.47; 9.88; 11.47; 14.83; 16.31; 17.19; 19.01; 20.88; 22.16. Even more specifically it may be characterised with the following x-ray powder diffraction peaks: 2θ (±0.2° 2θ): 4.94; 7.41; 8.31; 9.47; 9.88; 11.47; 13.19; 14.83; 15.65; 16.31; 17.19; 17.54; 18.26; 19.01; 19.23; 19.84; 20.18; 20.88; 22.16; 23.13; 23.58; 24.38; 24.81; 25.52; 25.87; 26.79; 27.32; 28.57; 29.07; 29.57; 30.08; 30.58; 30.97; 31.57; 32.18; 32.93; 33.75. Its characteristic x-ray powder diffractogram is shown in figure 5, and the 1% or higher intensity signals are summarised in the following table 3:

**Table 3**

| The x-ray powder diffraction data of the palbociclib hydrogen chloride (1:1) salt Form E (relative intensities ≥ 1%) | | | |
|---|---|---|---|
| Peak | 2θ (°) | d (Å) | Relative intensity (%) |
| 1 | 4.94 | 17.89 | 10 |
| 2 | 7.41 | 11.92 | 4 |
| 3 | 8.31 | 10.64 | 3 |
| 4 | 9.47 | 9.33 | 7 |
| 5 | 9.88 | 8.95 | 14 |
| 6 | 11.47 | 7.71 | 24 |
| 7 | 13.19 | 6.71 | 5 |
| 8 | 14.83 | 5.97 | 14 |
| 9 | 15.65 | 5.66 | 1 |
| 10 | 16.31 | 5.43 | 13 |
| 11 | 17.19 | 5.15 | 13 |
| 12 | 17.54 | 5.05 | 2 |
| 13 | 18.26 | 4.85 | 3 |
| 14 | 19.01 | 4.66 | 36 |
| 15 | 19.23 | 4.61 | 9 |
| 16 | 19.84 | 4.47 | 3 |
| 17 | 20.18 | 4.40 | 1 |
| 18 | 20.88 | 4.25 | 18 |
| 19 | 22.16 | 4.01 | 100 |
| 20 | 23.13 | 3.84 | 5 |
| 21 | 23.58 | 3.77 | 8 |
| 22 | 24.38 | 3.65 | 4 |
| 23 | 24.81 | 3.59 | 3 |
| 24 | 25.52 | 3.49 | 11 |
| 25 | 25.87 | 3.44 | 12 |
| 26 | 26.79 | 3.33 | 13 |
| 27 | 27.32 | 3.26 | 7 |
| 28 | 28.57 | 3.12 | 3 |
| 29 | 29.07 | 3.07 | 1 |
| 30 | 29.57 | 3.02 | 1 |
| 31 | 30.08 | 2.97 | 1 |
| 32 | 30.58 | 2.92 | 1 |
| 33 | 30.97 | 2.88 | 8 |
| 34 | 31.57 | 2.83 | 1 |
| 35 | 32.18 | 2.78 | 2 |
| 36 | 32.93 | 2.72 | 1 |
| 37 | 33.75 | 2.65 | 2 |

The water sorption isotherms of the palbociclib hydrogen chloride (1:1) salt Form E disclosed herein recorded at 25 °C are presented in figure 6. It is clearly visible that the substance advantageously does not exhibit significant hygroscopicity.

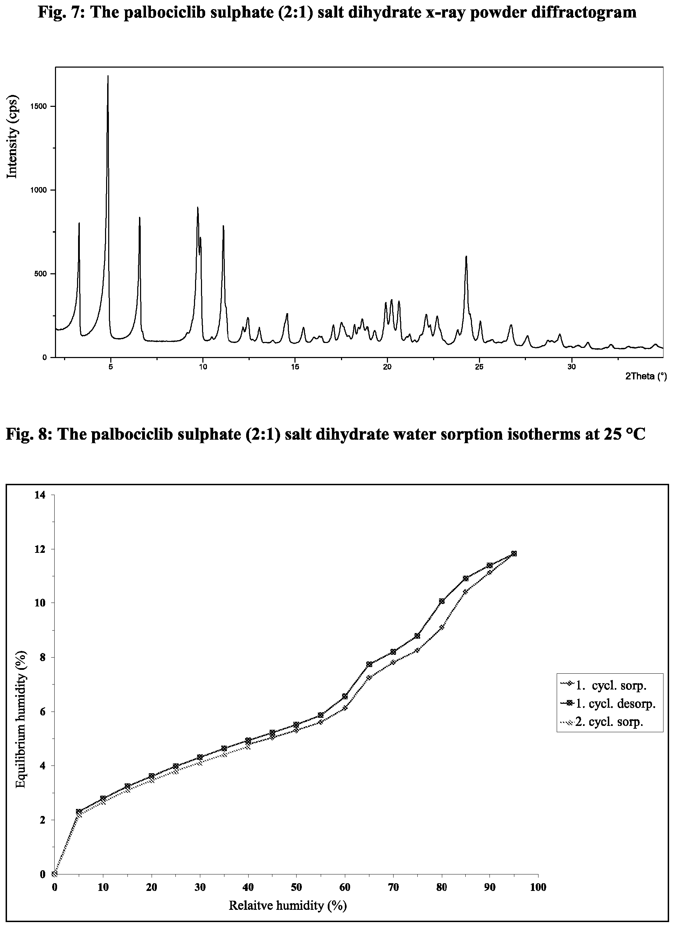

Disclosed herein is also the crystalline palbociclib sulphate (2:1) dihydrate salt form, the characteristic x-ray powder diffraction peaks of which are the following: 2θ (±0.2° 2θ): 3.29; 4.85; 6.56; 9.69; 11.09. More specifically it may be characterised with the following x-ray powder diffraction peak: 2θ (±0.2 °2θ): 3.29; 4.85; 6.56; 9.69; 9.84; 11.09; 11.24; 14.57; 20.61; 24.28. Even more specifically it may be characterised with the following x-ray powder diffraction peaks: 2θ (±0.2 °2θ): 3.29; 4.85; 6.56; 9.69; 9.84; 11.09; 11.24; 12.17; 12.42; 13.05; 14.41; 14.57; 15.45; 17.07; 17.48; 18.22; 18.41; 18.62; 18.91; 19.30; 19.89; 20.20; 20.61; 21.22; 22.10; 22.31; 22.69; 22.86; 23.80; 24.28; 24.49; 25.05; 26.69; 27.61; 29.36. Its characteristic x-ray powder diffractogram is shown in figure 7, and the intensity signals greater than 2% are summarised in the following table 4:

**Table 4**

| The x-ray powder diffraction data of the palbociclib sulphate (2:1) dihydrate salt (relative intensities > 2%) form | | | |
|---|---|---|---|
| Peak | 2θ (°) | d (Å) | Relative (%) |
| 1 | 3.29 | 26.87 | 62 |
| 2 | 4.85 | 18.21 | 100 |
| 3 | 6.56 | 13.46 | 43 |
| 4 | 9.69 | 9.12 | 29 |
| 5 | 9.84 | 8.98 | 27 |
| 6 | 11.09 | 7.97 | 30 |
| 7 | 11.24 | 7.87 | 8 |
| 8 | 12.17 | 7.27 | 3 |
| 9 | 12.42 | 7.12 | 6 |
| 10 | 13.05 | 6.78 | 4 |
| 11 | 14.41 | 6.14 | 3 |
| 12 | 14.57 | 6.08 | 7 |
| 13 | 15.45 | 5.73 | 4 |
| 14 | 17.07 | 5.19 | 5 |
| 15 | 17.48 | 5.07 | 4 |
| 16 | 18.22 | 4.86 | 5 |
| 17 | 18.41 | 4.81 | 3 |
| 18 | 18.62 | 4.76 | 5 |
| 19 | 18.91 | 4.69 | 5 |
| 20 | 19.30 | 4.60 | 3 |
| 21 | 19.89 | 4.46 | 9 |
| 22 | 20.20 | 4.39 | 10 |
| 23 | 20.61 | 4.31 | 11 |
| 24 | 21.22 | 4.18 | 3 |
| 25 | 22.10 | 4.02 | 7 |
| 26 | 22.31 | 3.98 | 4 |
| 27 | 22.69 | 3.92 | 7 |
| 28 | 22.86 | 3.89 | 3 |
| 29 | 23.80 | 3.74 | 3 |
| 30 | 24.28 | 3.66 | 22 |
| 31 | 24.49 | 3.63 | 6 |
| 32 | 25.05 | 3.55 | 6 |
| 33 | 26.69 | 3.34 | 6 |
| 34 | 27.61 | 3.23 | 3 |
| 35 | 29.36 | 3.04 | 4 |

The water sorption isotherms of the palbociclib sulphate (2:1) dihydrate salt form disclosed herein recorded at 25 °C are presented in figure 8. It is clearly visible that the substance exhibits significant hygroscopicity.

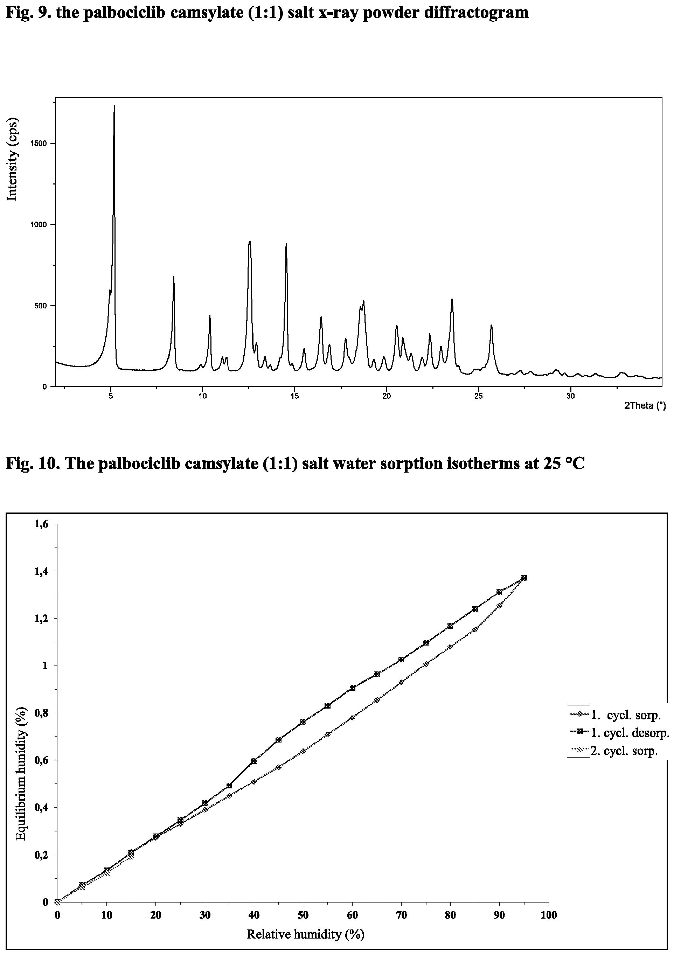

Disclosed herein is also the palbociclib camsylate (1:1) salt form, the characteristic x-ray powder diffraction peaks of which are the following: 2θ (±0.2° 2θ): 5.19; 8.42; 10.40; 12.58; 14.54. More specifically it may be characterised with the following x-ray powder diffraction peaks: 2θ (±0.2° 2θ): 4.95; 5.19; 8.42; 10.40; 12.58; 14.54; 16.45; 18.54; 18.73; 23.56. Even more specifically it may be characterised with the following x-ray powder diffraction peaks: 2θ (±0.2° 2θ): 4.95; 5.19; 8.42; 10.40; 11.06; 11.29; 12.58; 12.92; 13.39; 14.16; 14.54; 15.52; 16.45; 16.89; 17.74; 18.54; 18.73; 19.29; 19.84; 20.53; 20.87; 21.35; 21.90; 22.33; 22.92; 23.56; 25.69; 29.20. Its characteristic x-ray powder diffractogram is shown in figure 9, and the intensity signals greater than 4% are summarised in the following table 5:

**Table 5**

| The x-ray powder diffraction data of the palbociclib camsylate (1:1) salt form (relative intensities > 4%) | | | |
|---|---|---|---|
| Peak | 2θ (°) | d (Å) | Relative intensity (%) |
| 1 | 4.95 | 17.86 | 26 |
| 2 | 5.19 | 17.00 | 100 |
| 3 | 8.42 | 10.50 | 44 |
| 4 | 10.40 | 8.50 | 22 |
| 5 | 11.06 | 7.99 | 6 |
| 6 | 11.29 | 7.83 | 7 |
| 7 | 12.58 | 7.03 | 76 |
| 8 | 12.92 | 6.85 | 15 |
| 9 | 13.39 | 6.61 | 7 |
| 10 | 14.16 | 6.25 | 5 |
| 11 | 14.54 | 6.09 | 55 |
| 12 | 15.52 | 5.71 | 13 |
| 13 | 16.45 | 5.38 | 32 |
| 14 | 16.89 | 5.25 | 16 |
| 15 | 17.74 | 4.99 | 23 |
| 16 | 18.54 | 4.78 | 38 |
| 17 | 18.73 | 4.73 | 32 |
| 18 | 19.29 | 4.60 | 6 |
| 19 | 19.84 | 4.47 | 10 |
| 20 | 20.53 | 4.32 | 29 |
| 21 | 20.87 | 4.25 | 20 |
| 22 | 21.35 | 4.16 | 15 |
| 23 | 21.90 | 4.05 | 7 |
| 24 | 22.33 | 3.98 | 23 |
| 25 | 22.92 | 3.88 | 13 |
| 26 | 23.56 | 3.77 | 52 |
| 27 | 25.69 | 3.46 | 35 |
| 28 | 29.20 | 3.06 | 6 |

The water sorption isotherms of the palbociclib camsylate (1:1) salt disclosed herein recorded at 25 °C are presented in figure 10. It is clearly visible that the substance advantageously does not exhibit significant hygroscopicity.

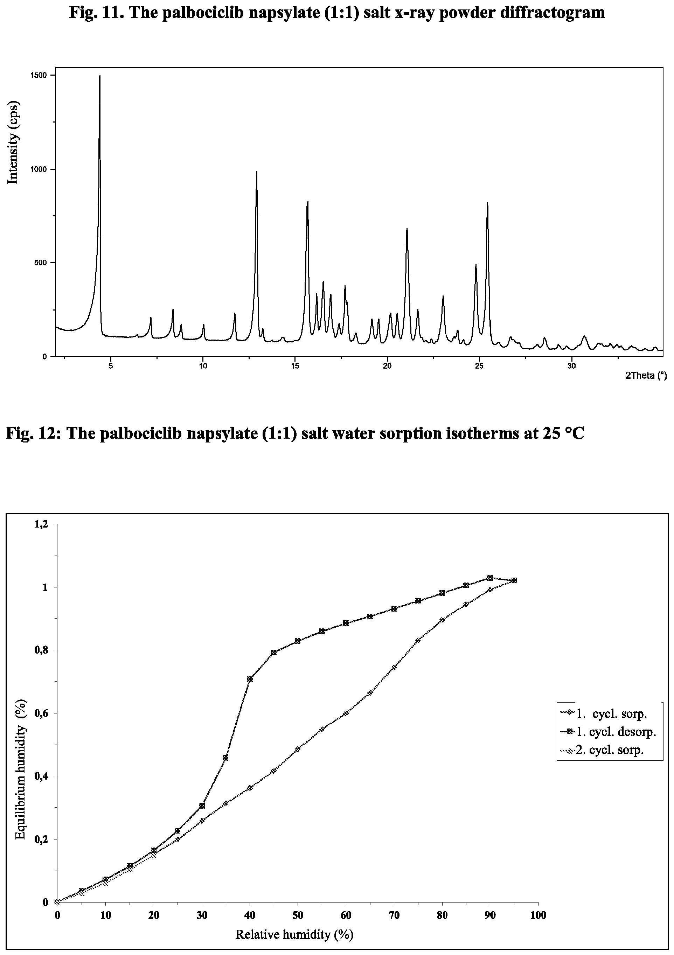

The subject of the invention more specifically is the palbociclib napsylate (1:1) salt form, the characteristic x-ray powder diffraction peaks of which are the following: 2θ (±0.2° 2θ): 4.41; 8.37; 12.90; 15.65; 25.45. More specifically it may be characterised with the following x-ray powder diffraction peak: 2θ (±0.2° 2θ): 4.41; 8.37; 11.76; 12.90; 15.65; 16.52; 16.90; 21.05; 24.79; 25.45. Even more specifically it may be characterised with the following x-ray powder diffraction peaks: 2θ (±0.2° 2θ): 4.41; 7.17; 8.37; 8.83; 10.04; 11.76; 12.90; 13.25; 15.65; 16.16; 16.52; 16.90; 17.38; 17.72; 17.82; 18.29; 19.13; 19.52; 20.15; 20.51; 21.05; 21.63; 22.39; 23.00; 23.80; 24.79; 25.45; 28.54; 29.28; 30.70; 32.12. Its characteristic x-ray powder diffractogram is shown in figure 11, and the intensity signals greater than 1% are summarised in the following table 6:

**Table 6**

| The x-ray powder diffraction data of the palbociclib napsylate (1:1) salt form (relative intensities > 1%) | | | |
|---|---|---|---|
| Peak | 2θ (°) | d (Å) | Relative intensity (%) |
| 1 | 4.41 | 20.00 | 100 |
| 2 | 7.17 | 12.31 | 7 |
| 3 | 8.37 | 10.56 | 11 |
| 4 | 8.83 | 10.01 | 5 |
| 5 | 10.04 | 8.81 | 6 |
| 6 | 11.76 | 7.52 | 10 |
| 7 | 12.90 | 6.86 | 59 |
| 8 | 13.25 | 6.68 | 4 |
| 9 | 15.65 | 5.66 | 55 |
| 10 | 16.16 | 5.48 | 15 |
| 11 | 16.52 | 5.36 | 26 |
| 12 | 16.90 | 5.24 | 22 |
| 13 | 17.38 | 5.10 | 8 |
| 14 | 17.72 | 5.00 | 16 |
| 15 | 17.82 | 4.97 | 13 |
| 16 | 18.29 | 4.85 | 6 |
| 17 | 19.13 | 4.64 | 10 |
| 18 | 19.52 | 4.54 | 7 |
| 19 | 20.15 | 4.40 | 16 |
| 20 | 20.51 | 4.33 | 11 |
| 21 | 21.05 | 4.22 | 57 |
| 22 | 21.63 | 4.11 | 14 |
| 23 | 22.39 | 3.97 | 2 |
| 24 | 23.00 | 3.86 | 25 |
| 25 | 23.80 | 3.74 | 7 |
| 26 | 24.79 | 3.59 | 35 |
| 27 | 25.45 | 3.50 | 63 |
| 28 | 28.54 | 3.13 | 6 |
| 29 | 29.28 | 3.05 | 2 |
| 30 | 30.70 | 2.91 | 12 |
| 31 | 32.12 | 2.78 | 2 |

The water sorption isotherms of the palbociclib napsylate (1:1) salt form disclosed herein recorded at 25 °C are presented in figure 12. It is clearly visible that the substance advantageously does not exhibit significant hygroscopicity.

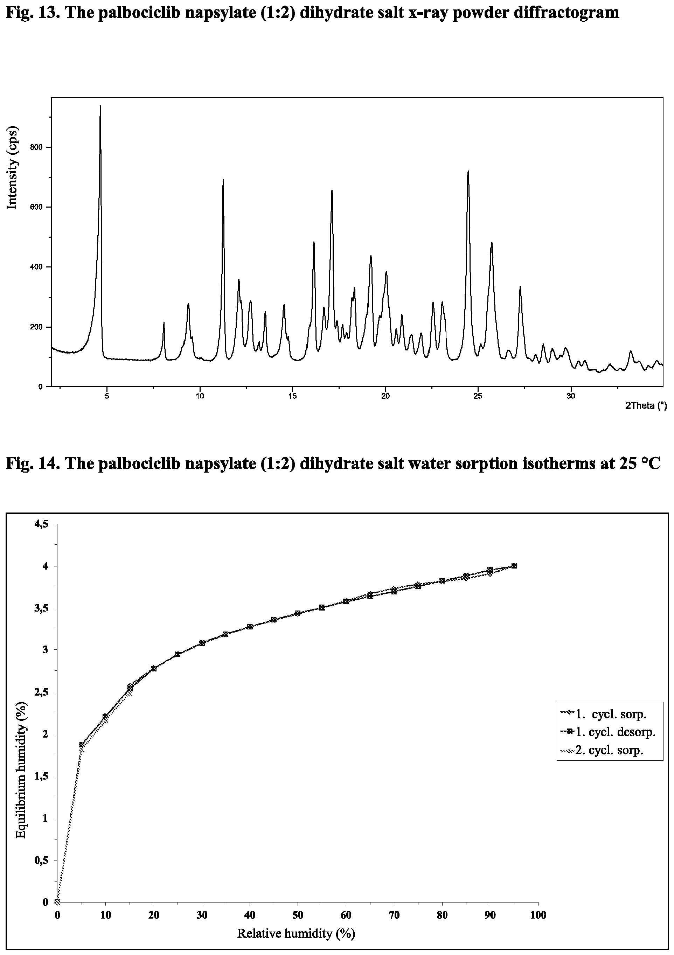

Disclosed herein is also the palbociclib napsylate (1:2) dihydrate salt form, the characteristic x-ray powder diffraction peaks of which are the following: 2θ (±0.2° 2θ): 4.66; 9.37; 11.25; 17.11; 24.46. More specifically it may be characterised with the following x-ray powder diffraction peaks: 2θ (±0.2° 2θ): 4.66; 8.06; 9.37; 11.25; 12.10; 12.70; 16.14; 17.11; 19.20; 24.46. Even more specifically it may be characterised with the following x-ray powder diffraction peaks: 2θ (±0.2° 2θ): 4.66; 8.06; 9.37; 9.56; 11.25; 12.10; 12.25; 12.70; 13.16; 13.52; 14.54; 14.77; 16.14; 16.68; 17.11; 17.38; 17.67; 17.91; 18.19; 18.33; 19.20; 19.62; 19.87; 20.03; 20.56; 20.87; 21.40; 21.92; 22.55; 23.04; 24.46; 25.13; 25.71; 26.56; 27.25; 28.13; 28.48; 28.96; 33.20. Its characteristic x-ray powder diffractogram is shown in figure 13, and the intensity signals greater than 4% are summarised in the following table 7:

**Table 7**

| The x-ray powder diffraction data of the palbociclib napsylate (1:2) dihydrate salt form (relative intensities > 4%) | | | |
|---|---|---|---|
| Peak | 2θ (°) | d (Å) | Relative intensity (%) |
| 1 | 4.66 | 18.93 | 100 |
| 2 | 8.06 | 10.96 | 9 |
| 3 | 9.37 | 9.43 | 24 |
| 4 | 9.56 | 9.25 | 11 |
| 5 | 11.25 | 7.86 | 45 |
| 6 | 12.10 | 7.31 | 32 |
| 7 | 12.25 | 7.22 | 9 |
| 8 | 12.70 | 6.97 | 29 |
| 9 | 13.16 | 6.72 | 5 |
| 10 | 13.52 | 6.54 | 16 |
| 11 | 14.54 | 6.09 | 22 |
| 12 | 14.77 | 5.99 | 14 |
| 13 | 16.14 | 5.49 | 39 |
| 14 | 16.68 | 5.31 | 17 |
| 15 | 17.11 | 5.18 | 64 |
| 16 | 17.38 | 5.10 | 11 |
| 17 | 17.67 | 5.02 | 9 |
| 18 | 17.91 | 4.95 | 8 |
| 19 | 18.19 | 4.87 | 15 |
| 20 | 18.33 | 4.84 | 20 |
| 21 | 19.20 | 4.62 | 57 |
| 22 | 19.62 | 4.52 | 19 |
| 23 | 19.87 | 4.47 | 15 |
| 24 | 20.03 | 4.43 | 29 |
| 25 | 20.56 | 4.32 | 9 |
| 26 | 20.87 | 4.25 | 20 |
| 27 | 21.40 | 4.15 | 15 |
| 28 | 21.92 | 4.05 | 16 |
| 29 | 22.55 | 3.94 | 27 |
| 30 | 23.04 | 3.86 | 37 |
| 31 | 24.46 | 3.64 | 87 |
| 32 | 25.13 | 3.54 | 5 |
| 33 | 25.71 | 3.46 | 51 |
| 34 | 26.56 | 3.35 | 9 |
| 35 | 27.25 | 3.27 | 38 |
| 36 | 28.13 | 3.17 | 5 |
| 37 | 28.48 | 3.13 | 11 |
| 38 | 28.96 | 3.08 | 9 |
| 39 | 29.66 | 3.01 | 7 |
| 40 | 33.20 | 2.70 | 9 |

The water sorption isotherms of the palbociclib napsylate (1:2) dihydrate salt Disclosed herein recorded at 25 °C are presented in figure 14. It is clearly visible that the substance exhibits significant hygroscopicity.

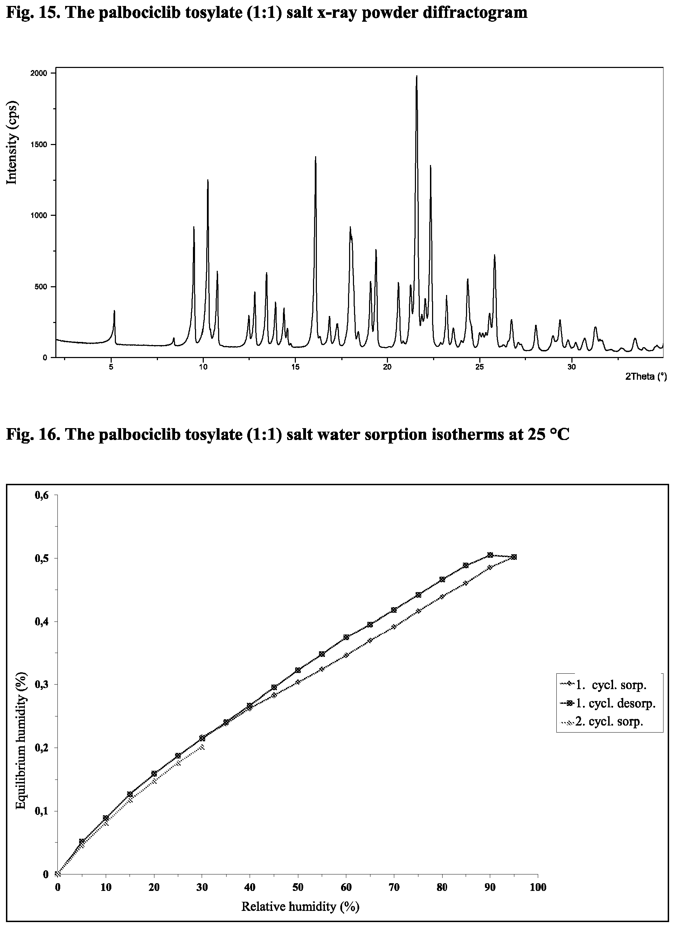

The subject of the invention more specifically is the palbociclib 4-toluenesulfonic acid (1:1) salt, the characteristic x-ray powder diffraction peaks of which are the following: 2θ (±0.2° 2θ): 5.18; 10.25; 13.45; 16.08; 21.56. More specifically it may be characterised with the following x-ray powder diffraction peaks: 2θ (±0.2° 2θ): 5.18; 9.49; 10.25; 10.76; 13.45; 16.08; 17.97; 19.39; 21.56; 22.33. Even more specifically it may be characterised with the following x-ray powder diffraction peaks: 2θ (±0.2° 2θ): 5.18; 8.40; 9.49; 10.25; 10.76; 12.48; 12.80; 13.45; 13.92; 14.37; 14.56; 16.08; 16.86; 17.27; 17.97; 18.39; 19.07; 19.39; 20.59; 21.25; 21.56; 22.03; 22.33; 23.21; 23.57; 24.37; 24.99; 25.55; 25.82; 26.72; 27.10; 28.05; 28.99; 29.35; 29.78; 30.21; 30.71; 31.26; 33.43. Its characteristic x-ray powder diffractogram is shown in figure 15, and the intensity signals greater than 1% are summarised in the following table 8:

**Table 8**

| The x-ray powder diffraction data of the palbociclib 4-toluenesulfonic acid (1:1) salt form (relative intensities > 1%) | | | |
|---|---|---|---|
| Peak | 2θ (°) | d (Å) | Relative intensity (%) |
| 1 | 5.18 | 17.06 | 10 |
| 2 | 8.40 | 10.52 | 2 |
| 3 | 9.49 | 9.31 | 36 |
| 4 | 10.25 | 8.62 | 55 |
| 5 | 10.76 | 8.22 | 25 |
| 6 | 12.48 | 7.09 | 9 |
| 7 | 12.80 | 6.91 | 17 |
| 8 | 13.45 | 6.58 | 23 |
| 9 | 13.92 | 6.36 | 14 |
| 10 | 14.37 | 6.16 | 14 |
| 11 | 14.56 | 6.08 | 4 |
| 12 | 16.08 | 5.51 | 65 |
| 13 | 16.86 | 5.25 | 10 |
| 14 | 17.27 | 5.13 | 11 |
| 15 | 17.97 | 4.93 | 42 |
| 16 | 18.39 | 4.82 | 4 |
| 17 | 19.07 | 4.65 | 25 |
| 18 | 19.39 | 4.57 | 30 |
| 19 | 20.59 | 4.31 | 22 |
| 20 | 21.25 | 4.18 | 18 |
| 21 | 21.56 | 4.12 | 100 |
| 22 | 22.03 | 4.03 | 29 |
| 23 | 22.33 | 3.98 | 57 |
| 24 | 23.21 | 3.83 | 19 |
| 25 | 23.57 | 3.77 | 9 |
| 26 | 24.37 | 3.65 | 31 |
| 27 | 24.99 | 3.56 | 6 |
| 28 | 25.55 | 3.48 | 16 |
| 29 | 25.82 | 3.45 | 31 |
| 30 | 26.72 | 3.33 | 11 |
| 31 | 27.10 | 3.29 | 3 |
| 32 | 28.05 | 3.18 | 9 |
| 33 | 28.99 | 3.08 | 7 |
| 34 | 29.35 | 3.04 | 13 |
| 35 | 29.78 | 3.00 | 5 |
| 36 | 30.21 | 2.96 | 3 |
| 37 | 30.71 | 2.91 | 3 |
| 38 | 31.26 | 2.86 | 11 |
| 39 | 31.53 | 2.84 | 5 |
| 40 | 33.43 | 2.68 | 6 |

The water sorption isotherms of the palbociclib 4-toluenesulfonic acid (1:1) salt according to the invention recorded at 25 °C are presented in figure 16. It is clearly visible that the substance advantageously does not exhibit significant hygroscopicity.

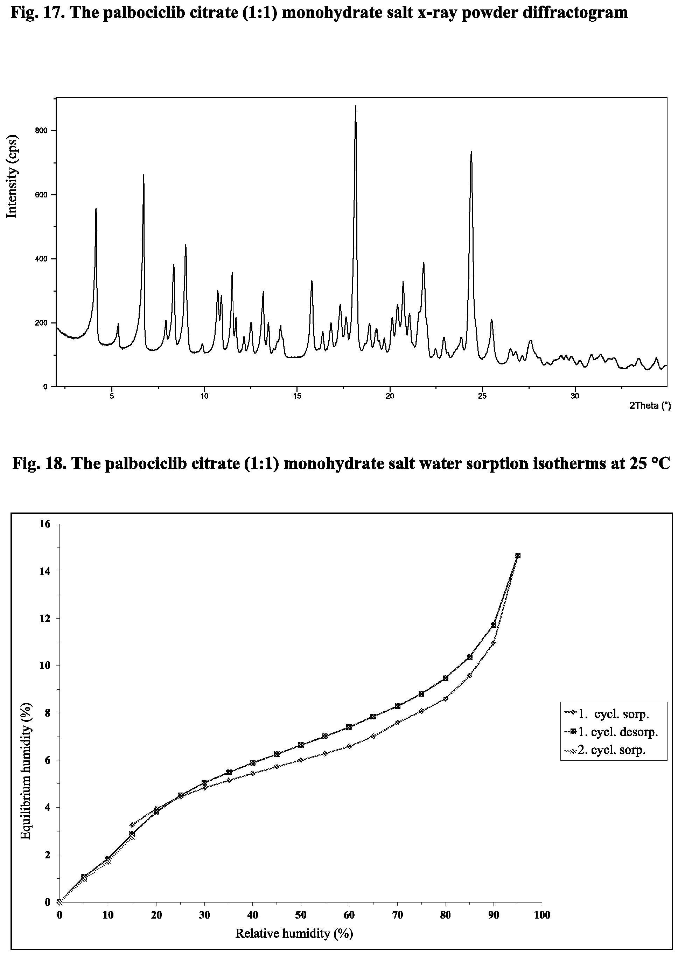

Disclosed herein is also the palbociclib citrate (1:1) monohydrate salt, the characteristic x-ray powder diffraction peaks of which are the following: 2θ (±0.2° 2θ): 4.16; 6.72; 8.33; 18.16; 24.40. More specifically it may be characterised with the following x-ray powder diffraction peaks: 2θ (±0.2° 2θ): 4.16; 6.72; 8.33; 8.98; 11.47; 13.17; 15.78; 18.16; 21.81; 24.40. Even more specifically it may be characterised with the following x-ray powder diffraction peaks: 2θ (±0.2° 2θ): 4.16; 5.34; 6.72; 7.91; 8.33; 8.98; 9.87; 10.71; 10.91; 11.47; 11.70; 12.14; 12.51; 13.17; 13.46; 14.10; 15.78; 16.40; 16.82; 17.31; 17.63; 18.16; 18.89; 19.25; 19.69; 20.11; 20.38; 20.69; 21.05; 21.81; 22.47; 22.92; 23.88; 24.40; 25.53; 26.50; 26.79; 27.17; 27.55. Its characteristic x-ray powder diffractogram is shown in figure 17, and the intensity signals greater than 2% are summarised in the following table 9:

**Table 9**

| The x-ray powder diffraction data of the palbociclib citrate (1:1) monohydrate salt form (relative intensities >2%) | | | |
|---|---|---|---|
| Peak | 2θ (°) | d (Å) | Relative intensity (%) |
| 1 | 4.16 | 21.21 | 52 |
| 2 | 5.34 | 16.53 | 8 |
| 3 | 6.72 | 13.15 | 59 |
| 4 | 7.91 | 11.17 | 9 |
| 5 | 8.33 | 10.61 | 31 |
| 6 | 8.98 | 9.84 | 46 |
| 7 | 9.87 | 8.96 | 3 |
| 8 | 10.71 | 8.26 | 19 |
| 9 | 10.91 | 8.10 | 19 |
| 10 | 11.47 | 7.71 | 25 |
| 11 | 11.70 | 7.56 | 12 |
| 12 | 12.14 | 7.29 | 5 |
| 13 | 12.51 | 7.07 | 13 |
| 14 | 13.17 | 6.72 | 27 |
| 15 | 13.46 | 6.57 | 10 |
| 16 | 14.10 | 6.28 | 9 |
| 17 | 15.78 | 5.61 | 31 |
| 18 | 16.40 | 5.40 | 12 |
| 19 | 16.82 | 5.27 | 15 |
| 20 | 17.31 | 5.12 | 24 |
| 21 | 17.63 | 5.03 | 12 |
| 22 | 18.16 | 4.88 | 100 |
| 23 | 18.89 | 4.69 | 14 |
| 24 | 19.25 | 4.61 | 13 |
| 25 | 19.69 | 4.50 | 8 |
| 26 | 20.11 | 4.41 | 14 |
| 27 | 20.38 | 4.35 | 26 |
| 28 | 20.69 | 4.29 | 31 |
| 29 | 21.05 | 4.22 | 18 |
| 30 | 21.81 | 4.07 | 41 |
| 31 | 22.47 | 3.95 | 5 |
| 32 | 22.92 | 3.88 | 12 |
| 33 | 23.88 | 3.72 | 19 |
| 34 | 24.40 | 3.65 | 93 |
| 35 | 25.53 | 3.49 | 28 |
| 36 | 26.50 | 3.36 | 8 |
| 37 | 26.79 | 3.33 | 6 |
| 38 | 27.17 | 3.28 | 3 |
| 39 | 27.55 | 3.23 | 16 |

The water sorption isotherms of the palbociclib citrate (1:1) monohydrate salt disclosed herein recorded at 25 °C are presented in figure 18. It is clearly visible that the substance exhibits significant hygroscopicity.

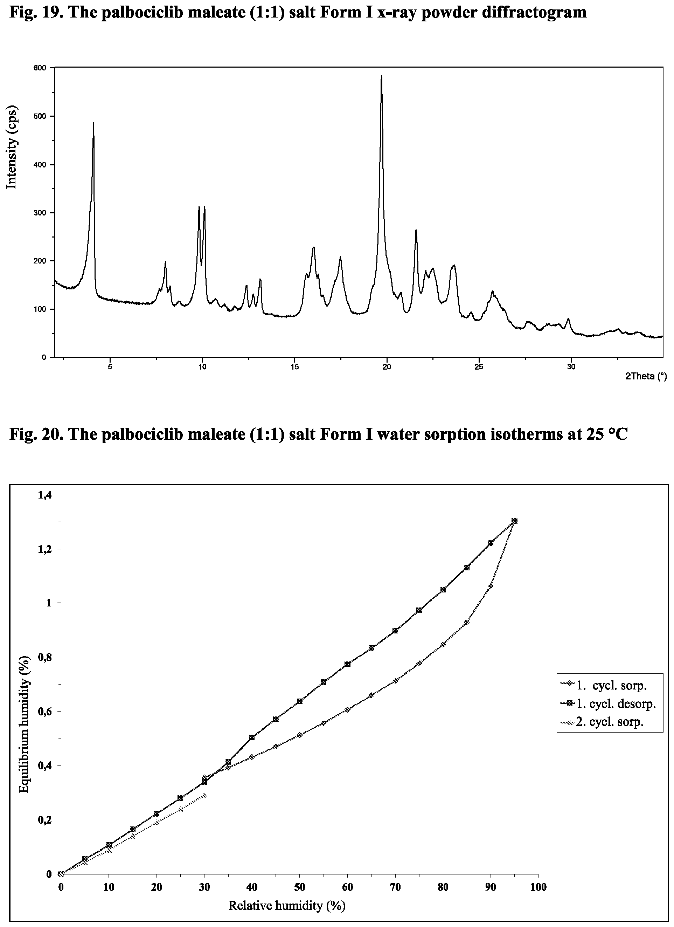

Disclosed herein is also the palbociclib maleate (Form I) (1:1) salt, the characteristic x-ray powder diffraction peaks of which are the following: 2θ (±0.2° 2θ): 4.12; 8.00; 9.81; 10.10; 19.70. More specifically it may be characterised with the following x-ray powder diffraction peaks: 2θ (±0.2° 2θ): 4.12; 8.00; 9.81; 10.10; 16.02; 17.46; 19.70; 21.56; 22.48; 23.63. Even more specifically it may be characterised with the following x-ray powder diffraction peaks: 2θ (±0.2° 2θ): 4.12; 7.71; 8.00; 8.25; 9.81; 10.10; 10.72; 12.41; 12.76; 13.14; 15.61; 16.02; 16.26; 16.51; 17.18; 17.46; 19.70; 21.56; 22.07; 22.48; 23.63; 24.59; 25.76; 27.61; 28.71; 29.30; 29.79. Its characteristic x-ray powder diffractogram is shown in figure 19, and the intensity signals greater than 3% are summarised in the following table 10:

**Table 10**

| The x-ray powder diffraction data of the palbociclib maleate (Form I) (1:1) salt form (relative intensities >3%) | | | |
|---|---|---|---|
| Peak | 2θ (°) | d (Å) | Relative intensity (%) |
| 1 | 4.12 | 21.45 | 57 |
| 2 | 7.71 | 11.45 | 10 |
| 3 | 8.00 | 11.04 | 17 |
| 4 | 8.25 | 10.71 | 9 |
| 5 | 9.81 | 9.01 | 33 |
| 6 | 10.10 | 8.75 | 33 |
| 7 | 10.72 | 8.24 | 10 |
| 8 | 12.41 | 7.13 | 10 |
| 9 | 12.76 | 6.93 | 7 |
| 10 | 13.14 | 6.73 | 11 |
| 11 | 15.61 | 5.67 | 17 |
| 12 | 16.02 | 5.53 | 30 |
| 13 | 16.26 | 5.45 | 13 |
| 14 | 16.51 | 5.36 | 12 |
| 15 | 17.18 | 5.16 | 21 |
| 16 | 17.46 | 5.07 | 29 |
| 17 | 19.70 | 4.50 | 100 |
| 18 | 21.56 | 4.12 | 32 |
| 19 | 22.07 | 4.02 | 20 |
| 20 | 22.48 | 3.95 | 66 |
| 21 | 23.63 | 3.76 | 44 |
| 22 | 24.59 | 3.62 | 8 |
| 23 | 25.76 | 3.46 | 22 |
| 24 | 27.61 | 3.23 | 5 |
| 25 | 28.71 | 3.11 | 6 |
| 26 | 29.30 | 3.05 | 4 |
| 27 | 29.79 | 3.00 | 6 |

The water sorption isotherms of the palbociclib maleate (Form I) (1:1) salt disclosed herein recorded at 25 °C are presented in figure 20. It is clearly visible that the substance advantageously does not exhibit significant hygroscopicity.

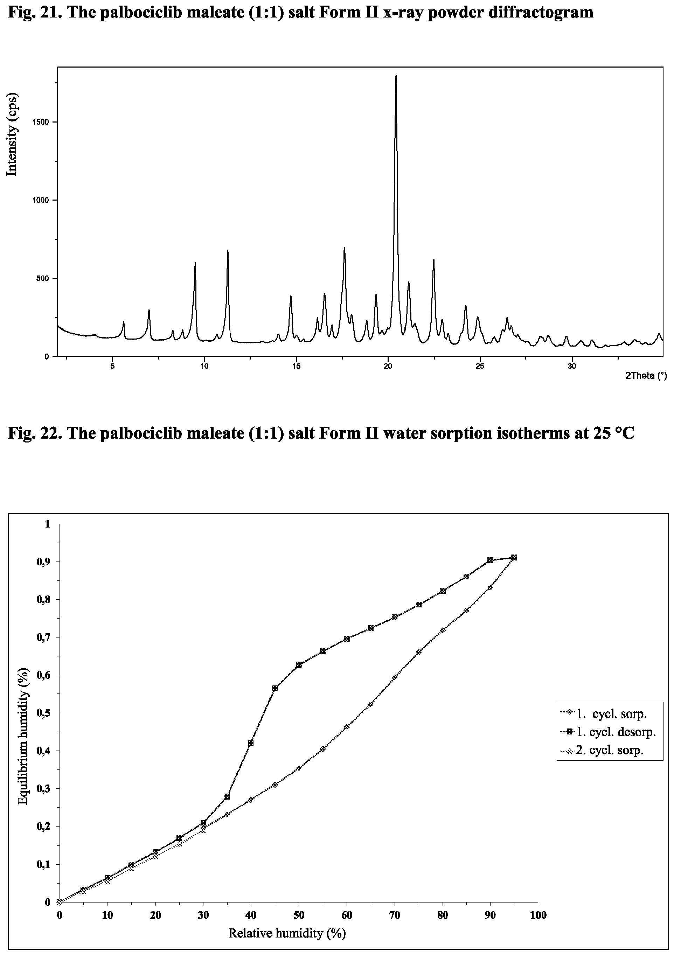

Disclosed herein is also the palbociclib maleate (Form II) (1:1) salt form, the characteristic x-ray powder diffraction peaks of which are the following: 2θ (±0.2° 2θ): 5.63; 7.00; 9.49; 11.27; 20.42. More specifically it may be characterised with the following x-ray powder diffraction peaks: 2θ (±0.2° 2θ): 5.63; 7.00; 9.49; 11.27; 14.71; 16.55; 17.60; 20.42; 21.10; 22.46. Even more specifically it may be characterised with the following x-ray powder diffraction peaks: 2θ (±0.2° 2θ): 5.63; 7.00; 8.28; 8.82; 9.49; 10.68; 11.27; 14.03; 14.71; 15.01; 16.15; 16.55; 16.93; 17.60; 18.03; 18.83; 19.34; 20.42; 21.10; 21.46; 22.46; 22.92; 23.27; 23.95; 24.23; 24.88; 25.79; 26.23; 26.47; 26.69; 27.07; 27.64; 28.26; 28.69; 29.67; 30.50; 31.08; 32.88; 34.74. Its characteristic x-ray powder diffractogram is shown in figure 21, and the intensity signals greater than 1% are summarised in the following table 11:

**Table 11**

| The x-ray powder diffraction data of the palbociclib maleate (Form II) (1:1) salt form (relative intensities >1%) | | | |
|---|---|---|---|
| Peak | 2θ (°) | d (Å) | Relative intensity (%) |
| 1 | 5.63 | 15.69 | 11 |
| 2 | 7.00 | 12.62 | 16 |
| 3 | 8.28 | 10.68 | 6 |
| 4 | 8.82 | 10.02 | 5 |
| 5 | 9.49 | 9.31 | 40 |
| 6 | 10.68 | 8.28 | 3 |
| 7 | 11.27 | 7.85 | 40 |
| 8 | 14.03 | 6.31 | 4 |
| 9 | 14.71 | 6.02 | 19 |
| 10 | 15.01 | 5.90 | 2 |
| 11 | 16.15 | 5.48 | 9 |
| 12 | 16.55 | 5.35 | 19 |
| 13 | 16.93 | 5.23 | 6 |
| 14 | 17.60 | 5.03 | 31 |
| 15 | 18.03 | 4.92 | 10 |
| 16 | 18.83 | 4.71 | 9 |
| 17 | 19.34 | 4.59 | 18 |
| 18 | 20.42 | 4.35 | 100 |
| 19 | 21.10 | 4.21 | 21 |
| 20 | 21.46 | 4.14 | 6 |
| 21 | 22.46 | 3.95 | 32 |
| 22 | 22.92 | 3.88 | 9 |
| 23 | 23.27 | 3.82 | 4 |
| 24 | 23.95 | 3.71 | 4 |
| 25 | 24.23 | 3.67 | 14 |
| 26 | 24.88 | 3.58 | 9 |
| 27 | 25.79 | 3.45 | 4 |
| 28 | 26.23 | 3.39 | 5 |
| 29 | 26.47 | 3.36 | 10 |
| 30 | 26.69 | 3.34 | 6 |
| 31 | 27.07 | 3.29 | 3 |
| 32 | 27.64 | 3.23 | 4 |
| 33 | 28.26 | 3.16 | 3 |
| 34 | 28.69 | 3.11 | 4 |
| 35 | 29.67 | 3.01 | 4 |
| 36 | 30.50 | 2.93 | 2 |
| 37 | 31.08 | 2.88 | 3 |
| 38 | 32.88 | 2.72 | 5 |
| 39 | 33.43 | 2.68 | 5 |
| 40 | 34.74 | 2.58 | 5 |

The water sorption isotherms of the palbociclib maleate (Form II) (1:1) salt disclosed herein recorded at 25 °C are presented in figure 22. It is clearly visible that the substance advantageously does not exhibit significant hygroscopicity.

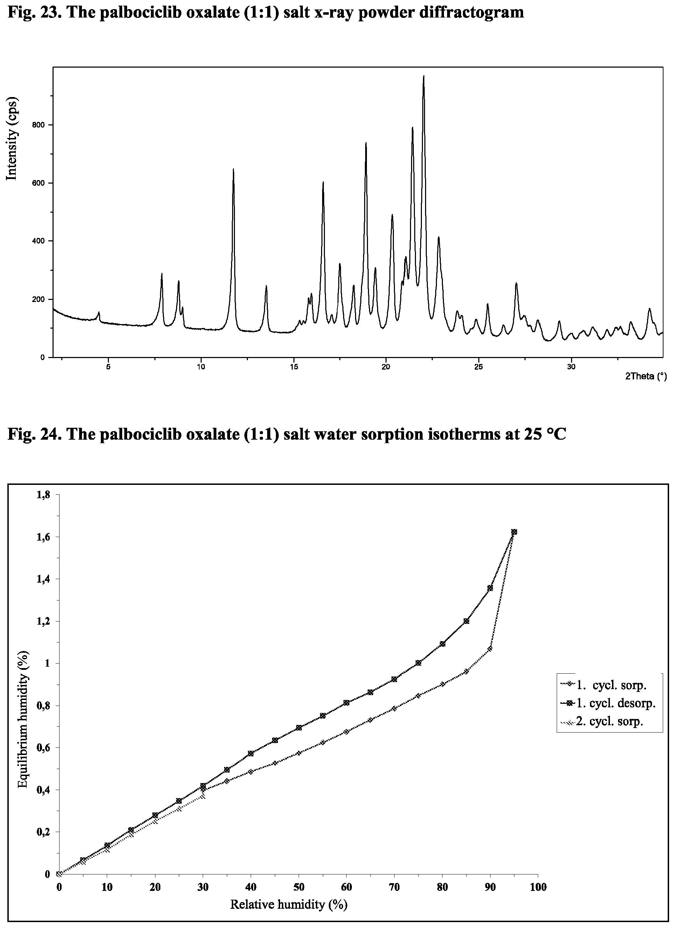

Disclosed herein is also the palbociclib oxalate (1:1) salt form, the characteristic x-ray powder diffraction peaks of which are the following: 2θ (±0.2° 2θ): 7.88; 11.75; 13.53; 16.59; 22.00. More specifically it may be characterised with the following x-ray powder diffraction peaks: 2θ (±0.2° 2θ): 7.88; 8.79; 11.75; 13.53; 16.59; 17.48; 18.91; 20.34; 21.41; 22.00. Even more specifically it may be characterised with the following x-ray powder diffraction peaks: 2θ (±0.2° 2θ): 4.49; 7.88; 8.79; 9.00; 11.75; 13.53; 15.30; 15.78; 15.94; 16.59; 17.04; 17.48; 18.27; 18.91; 19.43; 20.34; 20.85; 21.04; 21.41; 22.00; 22.83; 23.81; 24.09; 24.84; 25.48; 26.32; 27.02; 27.46; 27.76; 28.17; 29.33; 29.97; 30.64; 31.13; 31.91; 32.36; 32.64; 33.21; 34.22. Its characteristic x-ray powder diffractogram is shown in figure 23, and the intensity signals greater than 1% are summarised in the following table 12:

**Table 12**

| The x-ray powder diffraction data of the palbociclib oxalate (1:1) salt form (relative intensities >1%) | | | |
|---|---|---|---|
| Peak | 2θ (°) | d (Å) | Relative intensity (%) |
| 1 | 4.49 | 19.65 | 3 |
| 2 | 7.88 | 11.22 | 16 |
| 3 | 8.79 | 10.05 | 16 |
| 4 | 9.00 | 9.82 | 2 |
| 5 | 11.75 | 7.52 | 46 |
| 6 | 13.53 | 6.54 | 14 |
| 7 | 15.30 | 5.79 | 3 |
| 8 | 15.78 | 5.61 | 8 |
| 9 | 15.94 | 5.56 | 7 |
| 10 | 16.59 | 5.34 | 50 |
| 11 | 17.04 | 5.20 | 4 |
| 12 | 17.48 | 5.07 | 29 |
| 13 | 18.27 | 4.85 | 16 |
| 14 | 18.91 | 4.69 | 49 |
| 15 | 19.43 | 4.57 | 27 |
| 16 | 20.34 | 4.36 | 42 |
| 17 | 20.85 | 4.26 | 9 |
| 18 | 21.04 | 4.22 | 21 |
| 19 | 21.41 | 4.15 | 80 |
| 20 | 22.00 | 4.04 | 100 |
| 21 | 22.83 | 3.89 | 39 |
| 22 | 23.81 | 3.73 | 10 |
| 23 | 24.09 | 3.69 | 7 |
| 24 | 24.84 | 3.58 | 9 |
| 25 | 25.48 | 3.49 | 12 |
| 26 | 26.32 | 3.38 | 5 |
| 27 | 27.02 | 3.30 | 25 |
| 28 | 27.46 | 3.25 | 9 |
| 29 | 27.76 | 3.21 | 5 |
| 30 | 28.17 | 3.17 | 7 |
| 31 | 29.33 | 3.04 | 7 |
| 32 | 29.97 | 2.98 | 3 |
| 33 | 30.64 | 2.92 | 3 |
| 34 | 31.13 | 2.87 | 4 |
| 35 | 31.91 | 2.80 | 4 |
| 36 | 32.36 | 2.76 | 5 |
| 37 | 32.64 | 2.74 | 9 |
| 38 | 33.21 | 2.70 | 8 |
| 39 | 34.22 | 2.62 | 10 |

The water sorption isotherms of the palbociclib oxalate (1:1) salt disclosed herein recorded at 25 °C are presented in figure 24. It is clearly visible that the substance does not exhibit significant hygroscopicity.

A further subject of the invention relates to a method for the production of the palbociclib salts, characterised by that the above formula (**1**) palbociclib amorphous or crystalline, anhydrous, hydrate or solvate form is reacted with the desired organic or inorganic acid in a suitable organic solvent, then the salt formed is isolated.

The salts and salt polymorphs according to the invention may, on the one part, be produced by dissolving the palbociclib free base (**1**) in an organic solvent and reacting it with the desired acid at the appropriate temperature, the crystallized salt is isolated, and washed if necessary with organic solvent, then dried at the appropriate temperature.

The salts and salt polymorphs according to the invention may also be produced by reacting a suitable salt of palbociclib with a base under appropriate conditions, the crystallized salt is isolated, washed with an organic solvent if necessary, then dried at the appropriate temperature.

The salts and salt polymorphs according to the invention may also be produced by drying a suitable salt of palbociclib under suitable conditions, during which a solid phase morphological transformation takes place in the product and a substance with a crystalline structure different to that of the original is obtained.

The hydrate form of the salts according to the invention may, on the first part, also be produced by drying a suitable salt of palbociclib then by leaving it to stand in the air, and so it is made possible for the product to absorb water from the environment up until the stoichiometric amount is reached.

On the other part, the hydrate of the salts according to the invention may also be produced by performing salt formation in a mixture of a solvent miscible with water and water, and the crystallizing salt hydrate is isolated.

Any procedure used in the pharmaceuticals industry may be used for isolating the salt which serves for separating the solid phase and the liquid phase, for example, it may be filtered under atmospheric conditions, using vacuum filtration, or even under pressure, or even a centrifuge may be used as well.

Inorganic or organic acids may be used for the salt formation according to the invention, which may be the following: 4-toluenesulfonic acid.

The reaction may be performed in organic solvents, such as aliphatic alcohols with 1-6 carbon atoms, in straight-chain or ring ethers with 1-5 carbon atoms, in esters with 1-6 carbon atoms, in open-chain asymmetric or symmetric ketones, and in dipolar aprotic solvents, in addition mixtures of the listed solvents, as well as mixtures and blends of the listed solvents containing water.

1-4 carbon atom ethers, esters or alcohols, open-chain ketones or a dipolar aprotic solvents may be preferably used as the organic solvent for the performance of the reaction, especially preferable solvents include tetrahydrofuran, diethyl ether, ethyl acetate, acetonitrile, acetone, methyl alcohol, ethyl alcohol, 2-propanol, methyl ethyl ketone or mixtures of these, as well as mixtures and blends of the listed solvents containing water.

The formula (**1**) compound has more than one base centre, therefore the palbociclib salts described in the present invention may be formed with various forms of stoichiometry. Monosalts mean the ratio of the compound (**1**) and the acid component is 1:1; di-salts mean the ratio of the compound (**1**) and the acid component is 1:2.

The amount of acid used for forming the salt calculated for the amount of palbociclib (1) is preferably a 0.3-3.0 molar equivalent amount, more preferably a 0.5-2.5 molar equivalent amount.

A preferable process involves that an organic acid solution is used, and the salt formation reaction is performed at a temperature between 0 °C and the boiling point of the solvent or at the boiling point of the solvent.

An especially preferable process involves that the alcohol suspension or solution of palbociclib (**1**) and a 0.3-3.0 molar equivalent amount of the acid solution are reacted at a temperature close to the boiling point of the solvent. The product obtained from the reaction mixture is preferably obtained by filtration.

A preferable procedure involves that the acid is used in solid, crystalline state and the reaction is carried out at a temperature between 0 °C and the boiling point of the solvent or at the boiling point of the solvent.

An especially preferable process involves that the alcohol suspension or solution of palbociclib (1) and a 0.3-3.0 molar equivalent amount of the solid organic acid are reacted at a temperature close to the boiling point of the solvent. The product obtained from the reaction mixture is preferably obtained by filtration.

The new salts of palbociclib (**1**) according to the present invention may, on the one part, be produced by dissolving or suspending the palbociclib base (**1**) in a suitable solvent, preferably in a 1-6 carbon atom straight or branched chain alcohol, especially preferably in methanol, ethanol or 2-propanol, then 0.3-3.0 mol, preferably 0.5-2.5 mol acid is added to it on its own or in the form of a solution at a temperature between 0 °C and the reflux temperature of the solvent. If the salt precipitates at the temperature during adding or when cooled, after the required crystallization time the product is filtered, washed and dried. If the salt does not precipitate, the crystallization is initiated with a seed crystal, then the precipitated crystals are filtered. Apart from this, the process may involve the solvent being evaporated in a vacuum, and the residual material being crystalized by adding a suitable solvent or solvent mixture, finally the crystalline material is filtered, washed and dried.

The new salts of palbociclib (**1**) according to the present invention may, on the other part, be also produced by dissolving or suspending the palbociclib base (**1**) in a suitable solvent, preferably in a 1-6 carbon atom straight or branched carbon chain alcohol or open chain asymmetric or symmetric ketone, or halogenated hydrocarbon, especially preferably methanol, ethanol, 2-propanol, acetone or dichloromethane, optionally in mixtures of these with water, then 0.3-3.0 mol, preferably 0.5-2.5 mol acid on its own or in the form of a solution is added at a temperature between 0 °C and the reflux temperature of the solvent. After the required crystallizing time the product is filtered, washed and dried.

The production of the palbociclib hydrogen bromide (1:1) salt form as disclosed herein may involve that the palbociclib free base (**1**) is stirred in a straight chain alcohol type solvent, most preferably ethanol, an aqueous HBr solution is added to the mixture at a temperature between 0 °C and the boiling point of the solvent, preferably between room temperature and 80 °C, most preferably at 80 °C and the reaction mixture is stirred further, preferably for 48 hours at room temperature. The precipitated crystals are filtered, washed and dried.

The production of the palbociclib hydrogen bromide (1:2) dihydrate salt form as disclosed herein may involve that the palbociclib free base **(1)** is stirred in a straight chain alcohol type solvent, most preferably ethanol, an aqueous HBr solution is added to the mixture at a temperature between 0 °C and the boiling point of the solvent, preferably between room temperature and 80 °C, most preferably at 80 °C and the reaction mixture is stirred further, preferably for 18 hours at room temperature. The precipitated crystals are filtered, washed and dried.

The production of the palbociclib hydrogen chloride (1:1) salt Form E as disclosed herein may involve that the palbociclib free base (**1**) is stirred in a straight chain alcohol type solvent, most preferably ethanol, a 1 M hydrochloric acid solution is added to the mixture at a temperature between 0 °C and the boiling point of the solvent, preferably between room temperature and 80 °C, most preferably at 80 °C and the reaction mixture is stirred further, preferably for 93 hours at room temperature. The precipitated crystals are filtered, washed and dried.

The production of the palbociclib sulphate (2:1) dihydrate salt form as disclosed herein may involve that the palbociclib free base (**1**) is stirred in a dipolar aprotic solvent, most preferably acetonitrile, a cc. H₂SO₄ solution is added to the mixture at a temperature between 0 °C and the boiling point of the solvent, preferably between 0 °C and room temperature, most preferably at 0 °C and the reaction mixture is stirred further, preferably for 48 hours at room temperature. The precipitated crystals are filtered, washed and dried.

The production of the palbociclib camsylate (1:1) salt form as disclosed herein may involve that the palbociclib free base (**1**) is stirred in open chain asymmetric or symmetric ketones, most preferably acetone, *1S*-(+)-camphor-10-sulfonic acid is added to the mixture at a temperature between 0 °C and the boiling point of the solvent, preferably between room temperature and 80 °C, most preferably at 56 °C and the reaction mixture is stirred further, preferably for 24 hours at room temperature. The precipitated crystals are filtered, washed and dried.

The production of the palbociclib napsylate (1:1) salt form as disclosed herein may involve that the palbociclib free base (**1**) is stirred in open chain asymmetric or symmetric ketones, most preferably acetone, naphthalene-2-sulfonic acid monohydrate is added to the mixture at a temperature between 0 °C and the boiling point of the solvent, preferably between room temperature and 80 °C, most preferably at 56 °C and the reaction mixture is stirred further, preferably for 48 hours at room temperature. The precipitated crystals are filtered, washed and dried.

The production of the palbociclib napsylate (1:2) dihydrate salt form as disclosed herein may involve that the palbociclib free base (**1**) is stirred in a mixture of an open chain asymmetric or symmetric ketone and a halogenated hydrocarbon, most preferably acetone and dichloromethane, naphthalene-2-sulfonic acid monohydrate is added to the mixture at a temperature between 0 °C and the boiling point of the solvent, preferably between room temperature and 80 °C, most preferably at 40 °C and the reaction mixture is stirred further, preferably for 48 hours at room temperature. The precipitated crystals are filtered, washed and dried.

The production of the palbociclib tosylate (1:1) salt form may preferably involve that the palbociclib free base (**1**) is stirred in an open chain asymmetric or symmetric ketone, most preferably acetone, 4-toluenesulfonic acid monohydrate is added to the mixture at a temperature between 0 °C and the boiling point of the solvent, preferably between room temperature and 80 °C, most preferably at 56 °C and the reaction mixture is stirred further, preferably for 48 hours at room temperature. The precipitated crystals are filtered, washed and dried. The substance obtained in this way is mixed for 48 hours at room temperature in a mixture of a straight chain alcohol solvent, most preferably ethanol, and water, then the precipitated crystals are filtered, washed and dried.

The production of the palbociclib citrate (1:1) monohydrate salt form as disclosed herein may involve that the palbociclib free base (**1**) is stirred in a straight carbon chain alcohol solvent, most preferably methanol, citric acid monohydrate is added to the mixture at a temperature between 0 °C and the boiling point of the solvent, preferably between room temperature and 80 °C, most preferably at 65 °C and the reaction mixture is stirred further, preferably for 48 hours at room temperature. The precipitated crystals are filtered, washed and dried.

The production of the palbociclib maleate (Form I) (1:1) salt form as disclosed herein may involve that the palbociclib free base (**1**) is stirred in a dipolar aprotic solvent, most preferably acetonitrile, maleic acid is added to the mixture at a temperature between 0 °C and the boiling point of the solvent, preferably between 0 °C and room temperature, most preferably at 80 °C and the reaction mixture is mixed further, preferably for 48 hours at room temperature. The precipitated crystals are filtered, washed and dried.

The production of the palbociclib maleate (Form II) (1:1) salt form as disclosed herein may involve that the palbociclib free base (1) is stirred in a straight carbon chain alcohol solvent, most preferably ethanol, maleic acid is added to the mixture at a temperature between 0 °C and the boiling point of the solvent, preferably between room temperature and 80 °C, most preferably at 80 °C and the reaction mixture is stirred further, preferably for 24 hours at room temperature. The precipitated crystals are filtered, washed and dried.

The production of the palbociclib oxalate (1:1) salt form as disclosed herein may involve that the palbociclib free base (**1**) is stirred in a straight carbon chain alcohol solvent, most preferably ethanol, oxalic acid is added to the mixture at a temperature between 0 °C and the boiling point of the solvent, preferably between room temperature and 80 °C, most preferably at 80 °C and the reaction mixture is stirred further, preferably for 24 hours at room temperature. The precipitated crystals are filtered, washed and dried.

The thermal stress test and the forced stability test essentially provide an accelerated model of the degradations taking place in the pharmaceutical preparation during storage. The results of these predict that the new palbociclib (**1**) salts according to the invention will be more stable in pharmaceutical preparations under forced stability conditions than the known salts described in the literature. This preferable characteristic of the palbociclib salts of the present invention is very important from the point of view of the formulation of the pharmaceutical preparation, its storage and the minimising of the damaging effects on the human body.

The stability of the new palbociclib salts forming the subject of the present application was subject to detailed testing. The references used were the palbociclib Form A base also to be found in the originator preparation Ibrance® and the isethionate salt Form B used by them for a long time in human pharmaceutical development.

It was surprising to experience that the palbociclib salts according to the present invention exhibit the same or greater stability during 3 weeks of storage performed under various forced conditions as compared to the palbociclib base and the isethionate salt. It was experienced that among the new salts produced by us, the palbociclib salt with 4-toluenesulfonic acid (1:1) salt was shown to be especially stable.

The results are presented in the following tables 13-16. The tables indicate the percentage amount of the contaminants and their change. As the measured data show, on the basis of the increase in the amount of the contaminants both the tosylate and the hydrochloride salt Form E are more stable than the Form A base when measured under several different conditions. An especially significant advantage of our own salts as opposed to the base is that while in the base the amount of "other unknown contaminants" significantly increases even after 3 weeks of storage, this amount in the hydrochloride and tosylate salts remains unchanged. As a strict limit of <0.10% relates to unknown contaminants on the basis of pharmaceuticals industry guidelines, in a given case after several years of storage, this degree of increase of the amount of contaminant is critical in the Form A base.

**Table 13. The stability test data of the reference palbociclib base Form A**

| | | **Starting sample** | **3 weeks** | | |
|---|---|---|---|---|---|
| **Contaminant name** | ***RT (min)*** | | **25 °C 90% RH** | **40 °C 70% RH** | **70 °C** |
| Known contaminant 1 | 1.85 | - | 0.03% | - | 0.03% |
| Known contaminant 2 | 1.97 | 0.04% | 0.04% | 0.05% | 0.05% |
| Unknown contaminant | 1.70 | 0.04% | 0.04% | 0.05% | 0.04% |
| Unknown contaminant | 2.56 | 0.05% | 0.07% | 0.04% | 0.06% |
| Unknown contaminant | 3.04 | - | 0.05% | 0.04% | 0.05% |
| Total unknown contaminant | | 0.09% | 0.16% | 0.13% | 0.15% |
| **Total contamination** | | **0.13%** | **0.23%** | **0.17%** | **0.23%** |
| **Water content [m/m%]** | | **0.23%** | **0.42%** | **0.41%** | **0.39%** |

**Table 14. The stability test data of the reference palbociclib isethionate Form B**

| | | **Starting sample** | **3 weeks** | | |
|---|---|---|---|---|---|
| **Contaminant name** | ***RT (min)*** | | **25 °C 90% RH** | **40 °C 70% RH** | **70 °C** |
| Known contaminant 1 | 1.85 | - | - | - | - |
| Known contaminant 2 | 1.97 | 0.09% | 0.09% | 0.10% | 0.09% |
| Unknown contaminant | 1.70 | 0.03% | 0.03% | 0.03% | 0.03% |
| Unknown contaminant | 2.56 | 0.14% | 0.16% | 0.14% | 0.16% |
| Unknown contaminant | 1.36 | 0.03% | - | - | - |
| Total unknown contaminant | | 0.20% | 0.19% | 0.17% | 0.19% |
| **Total contamination** | | **0.29%** | **0.28%** | **0.27%** | **0.28%** |
| **Water content [m/m%]** | | **0.36%** | **0.97%** | **0.76%** | **0.83%** |

**Table 15. The stability test data of the new palbociclib hydrochloride (1:1) salt Form E disclosed herein**

| | | **Starting sample** | **3 weeks** | | |
|---|---|---|---|---|---|
| **Contaminant name** | ***RT (min)*** | | **25 °C 90% RH** | **40 °C 70% RH** | **70 °C** |
| Known contaminant 1 | 1.85 | - | - | - | - |
| Known contaminant 2 | 1.97 | 0.05% | 0.06% | 0.06% | 0.06% |
| Unknown contaminant | 1.70 | 0.05% | 0.06% | 0.06% | 0.06% |
| Unknown contaminant | 2.56 | 0.10% | 0.10% | 0.07% | 0.11% |
| Total unknown contaminant | | 0.15% | 0.16% | 0.13% | 0.17% |
| **Total contamination** | | **0.20%** | **0.22%** | **0.19%** | **0.23%** |
| **Water content [m/m%]** | | **0.21%** | **0.45%** | **0.41%** | **0.42%** |

**Table 16. The stability test data of the new palbociclib tosylate (1:1) salt according to the present application**

| | | **Starting sample** | **3 weeks** | | |
|---|---|---|---|---|---|
| **Contaminant name** | ***RT (min)*** | | **25 °C 90% RH** | **40 °C 70% RH** | **70 °C** |
| Known contaminant 1 | 1.85 | - | - | - | - |
| Known contaminant 2 | 1.97 | - | - | - | - |
| Unknown contaminant | 1.70 | 0.04% | 0.04% | 0.05% | 0.05% |
| Unknown contaminant | 2.56 | 0.04% | 0.05% | 0.04% | 0.05% |
| Total unknown contaminant | | 0.08% | 0.09% | 0.09% | 0.10% |
| **Total contamination** | | **0.08%** | **0.09%** | **0.09%** | **0.10%** |
| **Water content [m/m%]** | | **0.34%** | **0.51%** | **0.49%** | **0.49%** |

The measurement conditions (method, eluent, etc.) are the same as that described in the case of the purity test, see there.

In tables 13-16 it can also be seen that advantageously the palbococlib hydrochloride (1:1) Form E and tosylate (1:1) form exhibit significantly less water absorption under various conditions than the isethionate salt. the tosylate (1:1) salts may be preferably used for formulating pharmaceutical preparations both from the points of view of chemical stability and hygroscopicity.

However, oral formulation demands that the active substance be bio-available and that the degree of this bioavailability should not vary in a wide range. In the case of an orally administered preparation, this bioavailability may be influenced by numerous factors, such as the solubility and stability of the given medicine as well as its absorption in the gastrointestinal system.

The solubility of the palbociclib base in water strongly depends on the pH value of the aqueous medium. The normal pH value of the stomach varies between 1.2-1.8 [Remington: The Science and Practice of Pharmacy, 20th edition (2000, editor: A.R. Gennaro); chapter 32; C.J. Perigard: Clinical Analysis]. Patients suffering from cancer diseases also receive other medicines to relieve the side effects and other complaints, and so they may received antacids or proton pump inhibitors as well, which increase the pH value of the stomach.

The above justifies that the solubility experiments be performed at three different pH values (pH=1, pH=4.5 and pH=6.8).

The new palbociclib salts and salt polymorphs according to our invention are suitable for the production of pharmaceutical preparations that reduce the fluctuations occurring in the case of the bioavailability of the palbociclib base (1), therefore as opposed to the palbociclib base currently used in the Ibrance® preparation, the new palbociclib salts make better bioavailability possible.

### The determination of the solubility of the palbociclib salts, the performance of the measurement

### Preparation of the sample solution for the solubility measurement:

With analytical precision 500 mg salt form is measured into a 20.0 ml Erlenmeyer flask. 10 ml of medium is added, then shaken for 6 hours at a rate of 100 min⁻¹ at 37 °C. After shaking the solution is left to settle for 18 hours at 37 °C. The settled solution is filtered through a 0.45 µm filter. 1 ml of filtered solution is diluted with medium to 100.0 ml.

### The results of the solubility measurement:

| | | **Reference 1: Palbociclib base Form A** | **Reference 2: Palbociclib isethionate (1:1) Form B** | **Palbociclib hydrogen chloride (1:1) Form E (Reference example)** | **Palbociclib tosylate (1:1)** |
|---|---|---|---|---|---|
| | | **solubility (mg/ml)** | | | |
| | 0.1 M HCl | 50.7 | 99.5 | 32.1 | 20.0 |
| | pH=4.5 phosphate buffer | precipitated | precipitated | 2.1 | 0.2 |
| | pH=6.8 phosphate buffer | <0.03 | <0.03 | <0.03 | <0.03 |

From the summary table of the results it can be seen that at pH=1 the solubility data of the new salts forming the subject of our invention, i.e. palbociclib tosylate (1:1) are comparable to the solubility values of the reference palbociclib base Form A, while at pH-4.5 they are significantly better.

### Determination of the purity of the palbociclib salts

A UHPLC measurement method was used for determining the purity of the palbociclib salts according to the invention. A Waters Acquity UPLC device with an Acquity BEH C18. 1.7 µm, 2.1×50 mm column was used. The following purity data was obtained during the measurement process:

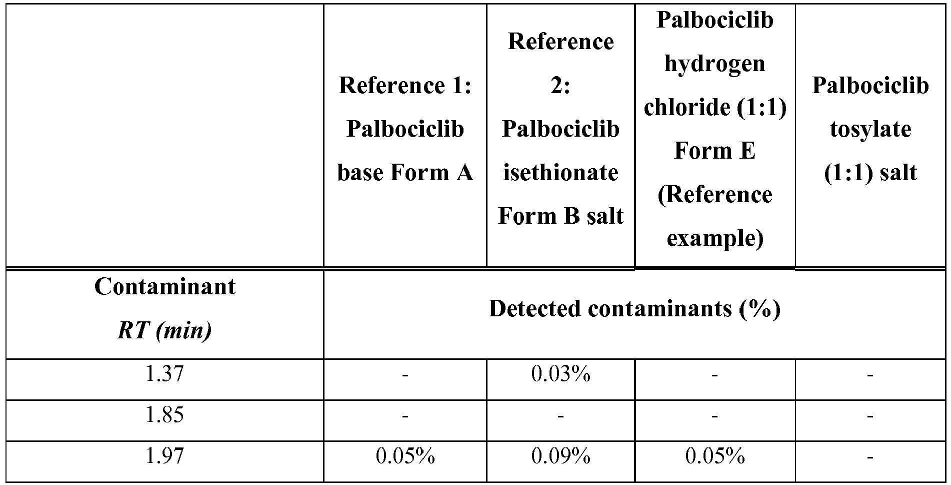

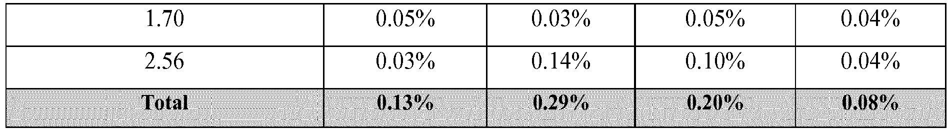

It can be seen from the purity data that the new palbociclib tosylate salt appearing in our invention may be produced at preferably greater purity as compared to the purity of the palbociclib base and isethionate salt used as references.

The subject of the invention also relates to a medical preparation containing a therapeutically effective amount of any of the palbociclib salts according to the invention and, optionally, one or more excipients used in the production of medicines. Furthermore, it relates to a method for the production of a medical preparation, characterised by that any palbociclib salt according to the invention is mixed with an appropriate amount of medically acceptable carrier and, optionally, other medically suitable excipients, and made into a galenic formulation.

The medical preparations according to the invention are preferably administered orally. Orally administered preparations include e.g. tablets, capsules, dragees, solutions, elixirs, suspensions and emulsions.

The medical preparations according to the invention may contain the usual medical carriers and/or excipients. Carriers include, for example, magnesium carbonate, magnesium stearate, talc, sugar, lactose, pectin, dextrin, starch, gelatine, tragacanth, methyl cellulose, sodium carboxymethyl cellulose, low melting point wax, PEG, cocoa butter, etc. In the case of capsules it is frequently the material of the capsule that serves as the carrier and so in such a case there is no need for a separate carrier. Orally administered preparations also include sachets and lozenges. Tablets, powders, capsules, pills, sachets and lozenges are especially suitable solid preparation forms.

The tablets may be produced by mixing the active substance with carrier materials with suitable characteristics in the appropriate ratio, and by pressing the desired shape and size tablets from the mixture.

Powders are produced by mixing finely powdered active substance and finely powdered carriers. Liquid preparations include solutions, suspensions and emulsions, from which, optionally, the active substance may be released after a delay. Aqueous or aqueous propylene glycol solutions are preferable. Liquid preparations serving for parenteral administration may be preferably produced in the form of an aqueous polyethylene glycol solution.

The medical preparations according to the invention are preferably produced in the form of dose units. The dose units contain the desired amount of active substance. The dose units may be distributed in packaged form, which contain separated amounts of the preparations (e.g. packaged tablets, capsules, powder in a vial or ampule). The dose unit relates to the capsule, tablet, sachet, lozenge as well as to the packaging containing a sufficient number of unit doses.

The subject of the invention also relates to a method for the production of the above medical preparations, in such a way that one of the palbociclib salts or a mixture of these is mixed with medically suitable solid or liquid diluents and/or excipients, and the mixture is placed into a galenic form.

The medical preparations according to the invention are produced using the usual methods of pharmaceutical production. If necessary, the medical preparations according to the invention may also contain further medical active substances with the compounds or a mixture of compounds according to the invention.

The subject of the invention also relates to the use of the compounds according to the present invention as a medical active substance.

The subject of the present invention relates to the use of any palbociclib salt according to the invention for the production of a medical preparation for the treatment of cancer diseases.

The subject of the invention also relates to the use of any palbociclib salt according to the invention for the treatment of cancer diseases. Cancer diseases include: advanced stage breast cancer, ER⁺/HER⁻ type breast cancer.

The advantage of the invention is that the compounds according to the present invention are preferable crystalline substances with homogenous morphology. Accordingly these substances have preferable and reproducible characteristics in terms of dissolution rate, bioavailability, chemical stability, hygroscopicity and processability (filtering, drying, tableting, etc.).

The active substances according to the invention may be produced with reproducible methods that can be easily realised even at the industrial scale.

The further details of the solution according to our invention are presented in the following examples, without limiting the scope of protection of the invention in any way to the examples mentioned.

### Examples

### Example 1 (reference example)

The production of palbociclib hydrogen bromide (1:1) salt 1.0 g (2.23 mmol) palbociclib base and 20 ml ethanol are measured into a device providing intensive stirring. While boiling 257.6 ml (2.23 mmol) of a 47% aqueous HBr solution is added to it drop by drop. The solution is cooled to room temperature, stirred for 48 hours, then this crystalline product is filtered, washed with a small amount of cold ethanol, then dried in a vacuum drying cabinet (40 °C, 13 mbar, 18 h).
Yield: 1.038 g (88.1%)
Mp.: no characteristic value, thermal decomposition takes place above 300 °C

Analysis with respect to the chemical formula C₂₄H₃₀BrN₇O₂ (528.46):

| | | | | |
|---|---|---|---|---|
| Calculated | C: 54.55 % | H: 5.72 % | N: 18.55 % | Br: 15.12 % |
| Measured | C: 54.46 % | H: 5.80 % | N: 18.05 % | Br: 14.77 % |

¹H-NMR (DMSO-*d*₆, 400 MHz): 10.26 (bs, 1H), 8.98 (s, 1H), 8.81 (b, 2H), 8.12 (d, J=2.8 Hz, 1H), 7.91 (d, J=9.0 Hz, 1H), 7.57 (dd, J1=2.8 Hz, J2=9.1 Hz, 1H), 5.84 (m, 1H), 3.44 (m, 8H), 2.43 (s, 3H), 2.32 (s, 3H), 2.24 (m, 2H), 1.90 (m, 2H), 1.78 (m, 2H), 1.59 (m, 2H).
¹³C-NMR (DMSO-*d*₆, 125 MHz): 202.62, 160.90, 158.61, 158.40, 154.94, 145.44, 142.47, 142.21, 135.99, 129.63, 126.05, 115.23, 106.97, 53.12, 45.83, 42.81, 31.49, 27.75, 25.32, 13.84.

### Example 2 (reference example)

The production of palbociclib hydrogen bromide (1:2) dihydrate salt
0.5 g (1.12 mmol) palbociclib base and 10 ml ethanol are measured into a device providing intensive stirring, then during heating at reflux 258.8 ml (2.24 mmol) of a 47% aqueous HBr solution is added to it drop by drop. The solution is cooled to room temperature, mixed for 18 hours, then this crystalline product is filtered, washed with a small amount of cold ethanol, then dried in a vacuum drying cabinet (40 °C, 12 mbar, 19 h).
Yield: 0.624 g (86.3%)
Mp.: 263-265 °C

Analysis with respect to the chemical formula C₂₄H₃₅Br₂N₇O₄ (645.40):

| | | | | |
|---|---|---|---|---|
| Calculated | C: 44.67 % | H: 5.47 % | N: 15.19 % | Br: 24.76 % |
| Measured | C: 44.67 % | H: 5.56 % | N: 14.91 % | Br: 24.26 % |

¹H-NMR (DMSO-*d*₆, 400 MHz): 11.34 (b, 1H), 9.04 (s, 1H), 8.91 (b, 2H), 8.06 (m, 2H), 7.77 (d, J=9.4 Hz, 1H), 5.87 (m, 1H), 3.46 (m, 4H), 3.29 (m, 4H), 2.45 (s, 3H), 2.37 (s, 3H), 2.21 (m, 2H), 1.95 (m, 2H), 1.81 (m, 2H), 1.60 (m, 2H).
¹³C-NMR (DMSO-*d*₆, 125 MHz): 202.38, 160.69, 157.34, 157.12, 155.21, 143.28, 142.32, 141.71, 131.99, 131.27, 127.68, 116.73, 108.76, 53.31, 45.27, 42.57, 31.46, 27.88, 25.49, 14.01.

### Example 3 (reference example)

The production of palbociclib hydrogen chloride (1:1) salt Form E
1.5 g (3.35 mmol) palbociclib base and 30 ml ethanol are measured into a device providing intensive stirring, then during boiling 3.18 ml (3.18 mmol) of a 1 M hydrochloric acid solution is added to it drop by drop. The solution is cooled to room temperature, mixed for 93 hours, then this crystalline product is filtered, washed with a small amount of cold ethanol and then MTBE, then dried in the air.
Yield: 0.62 g (90.8%)
Mp.: 288-290 °C

Analysis with respect to the chemical formula C₂₄H₃₀ClN₇O₂ (484.00):

| | | | | |
|---|---|---|---|---|
| Calculated | C: 59.56 % | H: 6.25 % | N: 20.26 % | Cl: 7.32 % |
| Measured | C: 59.40 % | H: 6.26 % | N: 20.00 % | Cl: 7.30 % |

¹H-NMR (DMSO-*d*₆, 400 MHz): 10.21 (bs, 1H), 9.27 (b, 2H), 8.97 (s, 1H), 8.12 (d, J=2.9 Hz, 1H), 7.92 (d, J=9.0 Hz, 1H), 7.54 (dd, J1=2.9 Hz, J2=9.1 Hz, 1H), 5.83 (m, 1H), 3.40 (m, 4H), 32.25 (m, 4H), 2.43 (s, 3H), 2.32 (s, 3H), 2.24 (m, 2H), 1.89 (m, 2H), 1.78 (m, 2H), 1.59 (m, 2H).
¹³C-NMR (DMSO-*d*₆, 125 MHz): 202.52, 160.87, 158.62, 158.37, 154.90, 145.45, 142.48, 142.16, 136.28, 129.53, 125.73, 115.11, 106.86, 53.11, 45.76, 42.61, 31.42, 27.70, 25.25, 13.76.

### Example 4 (reference example)

The production of palbociclib hydrogen chloride (1:1) salt Form E
1.0 g palbociclib hydrogen chloride (1:2) salt (1.92 mmol) produced using a method known from the literature, 20 ml ethanol and 268 µl triethylamine (1.92 mmol) are measured into a device providing intensive stirring. The suspension is stirred for 2 hours at 60 °C. The solution is cooled to room temperature, then this crystalline product is filtered, washed with a small amount of cold ethanol, then dried in the air.
Yield: 0.79 g (85.1 %)
Mp.: 288-290 °C

### Example 5 (reference example)

The production of palbociclib sulphate (C₂₄H₂₉N₇O₂ . ½ H₂SO₄ . 2 H₂O) dihydrate salt
1.0 g (2.23 mmol) palbociclib base and 20 ml acetonitrile are measured into a device providing intensive stirring, then during ice-water cooling 59.7 ml (1.12 mmol) of a solution of cc. H₂SO₄ prepared with 5 ml of acetonitrile is added to it drop by drop. The solution is cooled to room temperature, stirred for 48 hours, then the crystalline product is filtered, washed with a small amount of cold acetonitrile and then dried in a vacuum drying cabinet (50 °C, 15 mbar, 17 h).
Yield: 1.061 g (89.3%)
Mp.: no characteristic value, thermal decomposition takes place above 240 °C

Analysis with respect to the chemical formula C₄₈H₆₈N₁₄O₁₀S (1065.24):

| | | | | |
|---|---|---|---|---|
| Calculated | C: 54.12 % | H: 6.43 % | N: 18.41 % | S: 3.01 % |
| Measured | C: 54.66 % | H: 6.44 % | N: 18.43 % | S: 3.17 % |

¹H-NMR (DMSO-*d*₆, 400 MHz): 10.015 (bs, 1H), 8.96 (s, 1H), 8.08 (s, 1H), 7.88 (d, J=9.0 Hz, 1H), 7.49 (d, J=7.3 hz, 1H), 5.82 (m, 1H), 3.23 (m, 4H), 3.08 (m, 4H), 2.42 (s, 3H), 2.31 (s, 3H), 2.24 (m, 2H), 1.89 (m, 2H), 1.77 (m, 2H), 1.59 (m, 2H).
¹³C-NMR (DMSO-*d*₆, 125 MHz): 202.59, 160.92, 158.71, 158.40, 155.03, 143.24, 142.21, 139.83, 136.00, 129.49, 125.35, 115.30, 106.84, 53.11, 47.47, 44.02, 31.46, 27.72, 25.26, 13.78.

### Example 6 (reference example)

The production of palbociclib camsylate salt (1:1)
1.0 g (2.23 mmol) palbociclib base and 20 ml acetone are measured into a device providing intensive stirring, then during heating at reflux 558.2 mg (2.23 mmol) 1*S*-(+)-camphor-10-sulfonic acid is added. The solution is cooled to room temperature, stirred for 24 hours, then the crystalline product is filtered, washed with a small amount of cold acetone and then dried in the air.
Yield: 1.38 g (91.0%)
Mp.: no characteristic value, thermal decomposition takes place above 290 °C

Analysis with respect to the chemical formula C₃₄H₄₅N₇O₁₁S (679.84):

| | | | | |
|---|---|---|---|---|
| Calculated | C: 60.07 % | H: 6.67 % | N: 14.42 % | S: 4.72 % |
| Measured | C: 59.66 % | H: 6.66 % | N: 14.35 % | S: 4.76 % |

¹H-NMR (DMSO-*d*₆, 400 MHz): 10.21 (bs, 1H), 8.97 (s, 1H), 8.75 (b, 2H), 8.12 (d, J=2.8 Hz, 1H), 7.92 (d, J=9.0 Hz, 1H), 7.54 (dd, J1=2.9 Hz, J2=9.0 Hz, 1H), 5.83 (m, 1H), 3.39 (m, 4H), 3.27 (m, 4H), 2.88 (d, J=14.7 Hz, 1H), 2.67 (m, 1H), 2.43 (s, 3H), 2.39 (d, J=14.7 Hz, 1H), 2.32 (s, 3H), 2.24 (m, 3H), 1.93 (m, 1H), 1.86 (m, 1H), 1.80 (m, 1H), 1.79 (m, 2H), 1.77 (m, 2H), 1.59 (m, 2H), 1.28 (m, 2H), 1.05 (s, 3H), 0.74 (s, 3H).
¹³C-NMR (DMSO-*d*₆, 125 MHz): 216.51, 202.60, 160.90, 158.67, 158.43, 154.92, 145.50, 142.52, 142.21, 136.33, 129.55, 125.76, 115.17, 106.88, 58.41, 53.11, 47.23, 46.89, 45.92, 42.91, 42.43, 42.31, 31.47, 27.73, 26.57, 25.29, 24.32, 20.30, 19.73, 13.80.

### Example 7 (reference example)

The production of palbociclib napsylate (1:1) salt
1.0 g (2.23 mmol) palbociclib base and 25 ml acetone are measured into a device providing intensive stirring, then during heating at reflux 505.44 mg (2.23 mmol) naphthalene-2-sulfonic acid monohydrate is added. The solution is cooled to room temperature, stirred for 48 hours, then the crystalline product is filtered, washed with a small amount of cold acetone and then dried in a vacuum drying cabinet (55 °C, 6 mbar, 18 h).
Yield: 1.32 g (90.3%)
Mp.: no characteristic value, thermal decomposition takes place above 280 °C

Analysis with respect to the chemical formula C₃₄H₃₇N₇O₅S (655.77):

| | | | | |
|---|---|---|---|---|
| Calculated | C: 62.27 % | H: 5.69 % | N: 14.95 % | S: 4.89 % |
| Measured | C: 61.83 % | H: 5.67 % | N: 14.90 % | S: 4.85 % |

¹H-NMR (DMSO-*d*₆, 500 MHz): 10.17 (bs, 1H), 8.96 (s, 1H), 8.62 (b, 2H), 8.14 (s, 1H), 8.12 (d, J=2.8 Hz, 1H), 7.96 (m, 1H), 7.90 (m, 2H), 7.85 (d, J=8.6 Hz, 1H), 7.72 (dd, J1=1.5 Hz, J2=8.6 Hz, 1H), 7.54 (dd, J1=2.8 Hz, J2=9.0 Hz, 1H), 7.52 (m, 2H), 5.83 (m, 1H), 3.38 (m, 4H), 3.27 (m, 4H), 2.43 (s, 3H), 2.32 (s, 3H), 2.25 (m, 2H), 1.89 (m, 2H), 1.78 (m, 2H), 1.59 (m, 2H).
¹³C-NMR (DMSO-*d*₆, 125 MHz): 202.50, 160.86, 158.62, 158.34, 154.90, 154.86, 145.83, 145.47, 142.42, 142.14, 132.84, 132.29, 129.57, 128.56, 127.56, 127.38, 126.50, 126.38, 125.85, 124.14, 115.18, 106.90, 53.10, 45.86, 42.85, 31.42, 27.69, 25.24, 13.76.

### Example 8 (reference example)

The production of palbociclib napsylate (1:2) dihydrate salt
500 mg (1.12 mmol) palbociclib base, 20 ml dichloromethane and 5 ml acetone are measured into a device providing intensive stirring, then during heating at reflux 505.44 mg (2.23 mmol) naphthalene-2-sulfonic acid monohydrate is added. The solution is cooled to room temperature, stirred for 48 hours, then the crystalline product is filtered, washed with a small amount of cold MTBE and then dried in a vacuum drying cabinet (50 °C, 7 mbar, 18 h).
Yield: 0,849 g (87,7 %)
Mp.: 211-214 °C

Analysis with respect to the chemical formula C₄₄H₄₇N₇O₁₀S₂ (898.05):

| | | | | |
|---|---|---|---|---|
| Calculated | C: 58.85 % | H: 5.28% | N: 10.92 % | S: 7.14% |
| Measured | C: 58.88 % | H: 5.39% | N: 10.84 % | S: 7.12% |

¹H-NMR (DMSO-*d*₆, 400 MHz): 11.57 (b, 1H), 8.97 (s, 1H), 8.82 (b, 2H), 8.17 (bs, 2H), 8.12 (dd, J1=2.6 Hz, J2=9.5 Hz, 1H), 8.04 (d, J=2.8 Hz, 1H), 7.95 (m, 2H), 7.89 (m, 2H), 7.86 (d, J=8.7 Hz, 2H), 7.74 (dd, J1=1.6 Hz, J2=8.5 Hz, 2H), 7.68 (d, J=9.4 Hz, 1H), 7.51 (m, 4H), 5.86 (m, 1H), 3.45 (m, 4H), 3.32 (m, 4H), 2.46 (s, 3H), 2.32 (s, 3H), 2.20 (m, 2H), 1.97 (m, 2H), 1.82 (m, 2H), 1.59 (m, 2H).
¹³C-NMR (DMSO-*d*₆, 125 MHz): 202.32, 160.60, 156.92, 156.63, 155.20, 145.42, 142.74, 142.17, 141.53, 132.93, 132.28, 131.66, 128.62, 127.63, 127.60, 126.71, 126.54, 124.30, 124.03, 116.98, 109.14, 53.33, 45.13, 42.61, 31.44, 27.92, 25.56, 13.96.

### Example 9

The production of palbociclib tosylate (1:1) salt
1.0 g (2.23 mmol) palbociclib base, 20 ml acetone and 4 ml water are measured into a device providing intensive stirring, then during heating at reflux 424.2 mg (2.23 mmol) 4-toluenesulfonic acid monohydrate is added. The solution is cooled to room temperature, stirred for 48 hours, then the crystalline product is filtered, washed with a small amount of a cold acetone-water mixture and then dried in the air.

The substance obtained is stirred at room temperature in a mixture of 20 ml ethanol and 0.2 ml water, then the crystalline product is filtered, washed with a small amount of cold acetone and dried in the air.
Yield: 0.85 g (62.9%)
Mp.: no characteristic value, thermal decomposition takes place above 250 °C

Analysis with respect to the chemical formula C₃₁H₃₉N₇O₅S (621.76):

| | | | | |
|---|---|---|---|---|
| Calculated | C: 59.89 % | H: 6.32 % | N: 15.77 % | S: 5.16 % |
| Measured | C: 59.69 % | H: 6.08 % | N: 15.46 % | S: 5.69 % |

¹H-NMR (DMSO-*d*₆, 400 MHz): 10.26 (bs, 1H), 8.97 (s, 1H), 8.71 (b, 2H), 8.12 (d, J=2.8 Hz, 1H), 7.91 (d, J=9.1 Hz, 1H), 7.57 (dd, J1=2.9 Hz, J2=9.1 Hz, 1H), 7.48 (∼d, J=8.0 Hz, 2H), 7.12 (∼d, J=8.4 Hz, 2H), 5.83 (m, 1H), 3.37 (m, 4H), 3.29 (m, 4H), 2.43 (s, 3H), 2.32 (s, 3H), 2.29 (s, 3H), 2.25 (m, 2H), 1.89 (m, 2H), 1.78 (m, 2H), 1.59 (m, 2H).
¹³C-NMR (DMSO-*d*₆, 125 MHz): 202.62, 160.390, 158.62, 158.40, 154.94, 145.86, 145.45, 142.47, 142.21, 137.82, 135.98, 129.64, 128.25, 126.06, 125.67, 115.26, 106.98, 53.11, 45.87, 42.87, 31.49, 27.75, 25.32, 20.96, 13.84.

### Example 10 (reference example)

The production of palbociclib citrate (1:1) monohydrate salt
1.0 g (2.23 mmol) palbociclib base, 20 ml methanol and 0.1 ml water are measured into a device providing intensive stirring, then during heating at reflux 468.6 mg (2.23 mmol) citric acid monohydrate is added. The solution is cooled to room temperature, stirred for 48 hours, then the crystalline product is filtered, washed with a small amount of cold methanol then with MTBE and then dried in the air.
Yield: 1.20 g (83.9%)
Mp.: 192-196 °C

Analysis with respect to the chemical formula C₃₀H₃₇N₇O₉ (639.67):

| | | | |
|---|---|---|---|
| Calculated | C: 56.33 % | H: 5.83 % | N: 15.33 % |
| Measured | C: 56.12 % | H: 6.13 % | N: 15.97 % |

¹H-NMR (DMSO-*d*₆, 400 MHz): 10.18 (bs, 1H), 8.97 (s, 1H), 8.10 (d, J=2.9 Hz, 1H), 7.90 (d, J=9.0 hz, 1H), 7.52 (dd, J1=3.0 Hz, J2=9.1 Hz, 1H), 5.83 (m, 1H), 3.31 (m, 4H), 3.18 (m, 4H), 2.55 (d, J=15.5 Hz, 2H), 2.48 (d), 2.42 (s, 3H), 2.31 (s, 3H), 2.25 (m, 2H), 1.89 (m, 2H), 1.78 (m, 2H), 1.59 (m, 2H).
¹³C-NMR (DMSO-*d*₆, 500 MHz): 202.53, 177.25, 171.39, 160.89, 158.67, 158.37, 154.90, 145.24, 142.86, 142.18, 136.14, 129.50, 125.52, 115.21, 106.84, 71.24, 53.10, 46.72, 44.62, 43.43, 31.44, 27.70, 25.24, 13.75.

### Example 11 (reference example)

The production of palbociclib maleate (Form I) (1:1) salt
500 mg (1.12 mmol) palbociclib base and 10 ml acetonitrile are measured then during heating at reflux 130 mg (2.23 mmol) maleic acid is added. The solution is cooled to room temperature, stirred for 48 hours, then this crystalline product is filtered, washed with a small amount of cold acetonitrile and then dried in the air.
Yield: 0.585 g (92.7%)
Mp.: 198-200 °C

Analysis with respect to the chemical formula C₂₈H₃₃N₇O₆ (563.61):

| | | | |
|---|---|---|---|
| Calculated | C: 59.67 % | H: 5.90 % | N: 17.40 % |
| Measured | C: 59.20 % | H: 5.87 % | N: 17.17 % |

¹H-NMR (DMSO-*d*₆, 500 MHz): 10.15 (b, 1H), 8.96 (s, 1H), 8.12 (d, J=2.9 Hz, 1H), 7.92 (d, J=9.1 Hz, 1H), 7.54 (dd, J1=3.0 Hz, J2=9.1 Hz, 1H), 6.03 (s, 2H), 5.83 (m, 1H), 3.37 (m, 4H), 3.28 (m, 4H), 2.43 (s, 3H), 2.32 (s, 3H), 2.26 (m, 2H), 1.90 (m, 2H), 1.78 (m, 2H), 1.59 (m, 2H).
¹³C-NMR (DMSO-*d*₆, 500 MHz): 202.52, 167.31, 160.88, 158.64, 158.37, 154.90, 145.52, 142.44, 142.16, 136.33, 136.13, 129.54, 125.75, 115.15, 106.88, 53.11, 45.90, 42.85, 31.43, 27.70, 25.24, 13.77.

### Example 12 (reference example)

The production of palbociclib maleate (Form II) (1:1) salt
1000 mg (2.23 mmol) palbociclib base and 20 ml ethanol are measured into a device providing intensive stirring, then during heating at reflux 260 mg (2.23 mmol) maleic acid is added. The solution is cooled to room temperature, stirred for 24 hours, then the crystalline product is filtered, washed with a small amount of cold ethanol and then dried in the air.
Yield: 1.135 g (90.3%)
Mp.: 219-221 °C

Analysis with respect to the chemical formula C₂₈H₃₃N₇O₆ (563.61):

| | | | |
|---|---|---|---|
| Calculated | C: 59.67 % | H: 5.90 % | N: 17.40 % |
| Measured | C: 59.69 % | H: 5.96 % | N: 17.16 % |

¹H-NMR (DMSO-*d*₆, 500 MHz): 10.15 (b, 1H), 8.96 (s, 1H), 8.12 (d, J=2.9 Hz, 1H), 7.92 (d, J=9.1 Hz, 1H), 7.54 (dd, J1=3.0 Hz, J2=9.1 Hz, 1H), 6.03 (s, 2H), 5.83 (m, 1H), 3.37 (m, 4H), 3.28 (m, 4H), 2.43 (s, 3H), 2.32 (s, 3H), 2.26 (m, 2H), 1.90 (m, 2H), 1.78 (m, 2H), 1.59 (m, 2H).
¹³C-NMR (DMSO-*d*₆, 500 MHz): 202.52, 167.31, 160.88, 158.64, 158.37, 154.90, 145.52, 142.44, 142.16, 136.33, 136.13, 129.54, 125.75, 115.15, 106.88, 53.11, 45.90, 42.85, 31.43, 27.70, 25.24, 13.77.

### Example 13 (reference example)

The production of palbociclib oxalate (1:1) salt
1.5 g (3.35 mmol) palbociclib base and 30 ml ethanol are measured into a device providing intensive stirring, then during heating at reflux 0.302 g (3.35 mmol) oxalic acid is added. The solution is cooled to room temperature, stirred for 24 hours, then thise crystalline product is filtered, washed with a small amount of cold ethanol, then with MTBE and then dried in the air.
Yield: 1.03 g (57.2%)
Mp.: 245-247 °C

Analysis with respect to the chemical formula C₂₈H₃₃N₇O₆ (563.61):

| | | | |
|---|---|---|---|
| Calculated | C: 58.09 % | H: 5.81 % | N: 18.24 % |
| Measured | C: 57.96 % | H: 5.79 % | N: 17.87% |

¹H-NMR (DMSO-*d*₆, 400 MHz): 10.20 (bs, 1H), 8.97 (s, 1H), 8.12 (d, J=2.9 Hz, 1H), 7.91 (d, J=9.0 Hz, 1H), 7.54 (dd, J1=3.0 Hz, J2=9.1 Hz, 1H), 5.83 (m, 1H), 3.37 (m, 4H), 3.24 (m, 4H), 2.43 (s, 3H), 2.31 (s, 3H), 2.25 (m, 2H), 1.89 (m, 2H), 1.78 (m, 2H), 1.59 (m, 2H).
¹³C-NMR (DMSO-*d*₆, 500 MHz): 202.63, 163.29, 160.91, 158.68, 158.45, 154.93, 145.51, 142.53, 142.23, 136.34, 129.56, 125.77, 115.18, 106.89, 53.11, 45.92, 42.74, 31.49, 27.74, 25.31, 13.82.

## Claims

1. Crystalline salt forms of palbociclib formed with 4-toluenesulfonic acid.

2. The palbociclib 4-toluenesulfonic acid (1:1) salt form according to claim 1, the characteristic x-ray powder diffraction peaks of which are the following: 20 (±0.2 °2θ): 5.18; 10.25; 13.45; 16.08; 21.56.

3. Method for the production of the palbociclib salt according to claim 1 or 2, **characterised by** that palbociclib base is reacted in organic solvent or in a mixture of an organic solvent and water with an inorganic or organic acid, then the palbociclib salt formed is isolated, wherein the inorganic or organic acid is 4-toluenesulfonic acid.

4. The method according to claim 3, wherein the palbociclib salt formed is dried.

5. The method according to claim 4, **characterised by** that a 0.3-3.0 mol equivalent acid amount is used, preferably a 0.5-2.5 mol equivalent acid amount.

6. The method according to claim 4 or 5, **characterised by** that an aliphatic alcohol with 1-4 carbon atoms, an open chain symmetric or asymmetric ketone, a straight chain or ring ether with 1-5 carbon atoms, an ester with 1-6 carbon atoms or a dipolar aprotic solvent or halogenated hydrocarbon or mixtures of these is used as the organic solvent or mixtures and blends of these solvents formed with water is used for the performance of the reaction.

7. The method according to claim 6, **characterised by** that acetonitrile, methanol, ethanol, dichloromethane, acetone or mixtures of these, or mixtures and blends of these solvents formed with water is used as the solvent.

8. The method according to claims 3 to 7, **characterised by** that the reaction is performed at a temperature between 0°C and the boiling point of the solvent preferably at a temperature between room temperature and 80°C.

9. Medicinal preparation containing a palbociclib salt according to any of claims 1 to 2 and one or more excipients used in pharmaceutical formulation.

10. Palbociclib salt according to any of claims 1 to 2 for use in the treatment of cancer diseases.

11. The palbociclib salt for use according to claim 10, wherein the cancer disease is advanced stage breast cancer.

12. The palbociclib salt for use according to claim 11, wherein the cancer disease is an ER+/HER- breast cancer condition.

## Patentansprüche

1. Kristalline Salzformen von Palbociclib, gebildet mit 4-Toluolsulfonsäure.

2. Salzform der Palbociclib-4-Toluolsulfonsäure (1:1) nach Anspruch 1, deren charakteristische Peaks der Pulverröntgenbeugung die folgenden sind: 20 (±0.2°2θ): 5,18; 10,25; 13,45; 16,08; 21,56.

3. Verfahren zur Herstellung des Palbociclib-Salzes nach Anspruch 1 oder 2, **dadurch gekennzeichnet, dass** die Palbociclib-Base in einem organischen Lösungsmittel oder in einer Mischung aus einem organischen Lösungsmittel und Wasser mit einer anorganischen oder organischen Säure umgesetzt wird, wobei das gebildete Palbociclib-Salz isoliert ist, wobei die anorganische oder organische Säure 4-Toluolsulfonsäure ist.

4. Verfahren nach Anspruch 3, wobei das gebildete Palbociclib-Salz getrocknet wird.

5. Verfahren nach Anspruch 4, **dadurch gekennzeichnet, dass** eine 0,3-3,0 Moläquivalente Säuremenge, vorzugsweise eine 0,5-2,5 Moläquivalente Säuremenge, verwendet wird.

6. Verfahren nach Anspruch 4 oder 5, **dadurch gekennzeichnet, dass** ein aliphatischer Alkohol mit 1-4 Kohlenstoffatomen, ein offenkettiges symmetrisches oder asymmetrisches Keton, ein geradkettiger oder Ringether mit 1-5 Kohlenstoffatomen, ein Ester mit 1-6 Kohlenstoffatomen oder ein dipolares aprotisches Lösungsmittel oder ein halogenierter Kohlenwasserstoff oder Gemische davon als organisches Lösungsmittel oder Gemische verwendet werden, und Mischungen dieser mit Wasser gebildeten Lösungsmittel zur Durchführung der Reaktion verwendet werden.

7. Verfahren nach Anspruch 6, **dadurch gekennzeichnet, dass** Acetonitril, Methanol, Ethanol, Dichlormethan, Aceton oder Gemische davon, oder Gemische und Mischungen dieser mit Wasser gebildeten Lösungsmittel als Lösungsmittel verwendet werden.

8. Verfahren nach einem der Ansprüche 3 bis 7, **dadurch gekennzeichnet, dass** die Reaktion bei einer Temperatur zwischen 0°C und dem Siedepunkt des Lösungsmittels vorzugsweise bei einer Temperatur zwischen einer Raumtemperatur und 80°C durchgeführt wird.

9. Medizinisches Präparat, das ein Palbociclib-Salz gemäß einem der Ansprüche 1 bis 2 und einen oder mehrere in der pharmazeutischen Formulierung verwendete Hilfsstoffe enthält.

10. Palbociclib-Salz nach einem der Ansprüche 1 bis 2 zur Verwendung bei der Behandlung von Krebserkrankungen.

11. Palbociclib-Salz zur Verwendung nach Anspruch 10, wobei die Krebserkrankung Brustkrebs im fortgeschrittenen Stadium ist.

12. Palbociclib-Salz zur Verwendung nach Anspruch 11, wobei die Krebserkrankung eine ER+/HER- Brustkrebserkrankung ist.

## Revendications

1. Sel cristallin constitué de palbociclib formé avec de l'acide 4-toluènesulfonique.

2. Forme de sel d'acide 4-toluènesulfonique de palbociclib (1:1) selon la revendication 1, dont les pics caractéristiques de diffraction des rayons X sur poudre sont les suivants : 20 (±0,2°2θ): 5,18 ; 10,25 ; 13,45 ; 16,08 ; 21,56.

3. Procédé pour la production du sel de palbociclib selon la revendication 1 ou 2, **caractérisé en ce que** la base de palbociclib est faite réagir dans un solvant organique ou dans un mélange d'un solvant organique et eau avec un acide inorganique ou organique, puis le sel de palbociclib formé est isolé, dans lequel l'acide inorganique ou organique est de l'acide 4-toluènesulfonique.

4. Procédé selon la revendication 3, dans lequel le sel de palbociclib formé est séché.

5. Procédé selon la revendication 4, **caractérisé en ce qu'**une quantité d'acide équivalente à 0,3-3,0 mol est utilisée, de préférence une quantité d'acide équivalente à 0,5-2,5 mol.

6. Procédé selon la revendication 4 ou 5, **caractérisé en ce qu'**un alcool aliphatique avec 1-4 atomes de carbone, une cétone symétrique ou asymétrique à chaîne ouverte, un éther à chaîne droite ou cyclique avec 1-5 atomes de carbone, un ester avec 1-6 atomes de carbone ou un solvant aprotique dipolaire ou un hydrocarbure halogéné ou leurs mélanges est utilisé en tant que solvant organique ou des mélanges et mixtures de ces solvants formées avec de l'eau sont utilisées pour la réalisation de la réaction.

7. Procédé selon la revendication 6, **caractérisé en ce que** de l'acétonitrile, du méthanol, de l'éthanol, du dichlorométhane, de l'acétone ou leurs mélanges, ou des mélanges et mixtures de ces solvants formées avec de l'eau est utilisé en tant que solvant.

8. Procédé selon les revendications 3 à 7, **caractérisé en ce que** la réaction est réalisée à une température entre 0 °C et le point d'ébullition du solvant de préférence à une température entre la température ambiante et 80 °C.

9. Préparation médicale contenant un sel de palbociclib selon l'une quelconque des revendications 1 à 2 et un ou plusieurs excipients utilisés dans une formulation pharmaceutique.

10. Sel de palbociclib selon l'une quelconque des revendications 1 à 2 pour une utilisation dans le traitement de maladies cancéreuses.

11. Sel de palbociclib pour une utilisation selon la revendication 10, dans lequel la maladie cancéreuse est un stade avancé de cancer du sein.

12. Sel de palbociclib pour une utilisation selon la revendication 11, dans lequel la maladie cancéreuse est une condition de cancer du sein ER+/HER-.
